(19) Europäisches Patentamt
European Patent Office
Office européen des brevets

(11) **EP 3 877 034 B1**

(12) # EUROPEAN PATENT SPECIFICATION

(45) Date of publication and mention
of the grant of the patent:
**27.05.2026  Bulletin 2026/22**

(21) Application number: **19836105.7**

(22) Date of filing: **08.11.2019**

(51) International Patent Classification (IPC):
*A61M 31/00* (2006.01)     *A61M 25/01* (2006.01)
*A61M 25/04* (2006.01)

(52) Cooperative Patent Classification (CPC):
**A61M 31/002;** A61M 25/0102; A61M 25/0108;
A61M 25/04; A61M 2210/1082

(86) International application number:
**PCT/US2019/060493**

(87) International publication number:
**WO 2020/097476 (14.05.2020 Gazette 2020/20)**

(54) **DRUG DELIVERY DEVICES AND SYSTEMS FOR LOCAL DRUG DELIVERY TO THE UPPER URINARY TRACT**

WIRKSTOFFABGABEVORRICHTUNGEN UND SYSTEME ZUR LOKALEN WIRKSTOFFABGABE AN DEN OBEREN HARNTRAKT

DISPOSITIFS, SYSTÈMES ET PROCÉDÉS D'ADMINISTRATION DE MÉDICAMENT POUR L'ADMINISTRATION LOCALE DE MÉDICAMENT AU TRACTUS URINAIRE SUPÉRIEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:   **09.11.2018   US 201862757798 P**

(43) Date of publication of application:
**15.09.2021   Bulletin 2021/37**

(73) Proprietor: **Janssen Biotech, Inc.**
**Horsham, PA 19044 (US)**

(72) Inventors:
• **ABBATE, Emily**
  **Nashua, New Hampshire 03062 (US)**
• **DANIEL, Karen**
  **Newtonville, Massachusetts 02460 (US)**
• **CAULKINS, John**
  **Waltham, Massachusetts 02451 (US)**
• **HO DUC, Hong Linh**
  **Weston, Massachusetts 02493 (US)**
• **GREENAWAY, Erik**
  **Ayer, Massachusetts 01432 (US)**

(74) Representative: **Carpmaels & Ransford LLP**
**One Southampton Row**
**London WC1B 5HA (GB)**

(56) References cited:
WO-A1-2017/132372     US-A1- 2004 215 169
US-A1- 2016 287 369     US-A1- 2017 165 460

## Description

### Cross-Reference to Related Applications

This application claims priority benefit to U.S. Provisional Patent Application No. 62/757,798, filed November 9, 2018.

### Field of the Invention

This disclosure relates generally to drug delivery devices deployable *in vivo* for local controlled delivery of therapeutic and prophylactic agents to the upper urinary tract of patients in need thereof, and, more particularly to drug delivery devices and methods (not claimed) for local administration of drug into a patient's renal pelvis over an extended period.

### Background

Implantable drug delivery devices are known for targeted, e.g., local or regional, drug delivery in order to avoid one or more problems associated with systemic drug delivery. Targeted delivery of drug to some tissue sites, however, has significant room for improvement. Such sites include the kidneys and ureters.

Currently available drug delivery device-based treatments are invasive and/or are able to deliver drug only while a delivery catheter extends into the patient from outside the body. Some such systems, such as the BENEPHIT™ renal infusion system, deliver a therapeutic agent directly to the kidneys via the renal arteries through a catheter system, but undesirably require an arterial puncture in an interventional or surgical procedure.

There remains a need for less invasive drug delivery devices and methods to provide drug delivery into the upper urinary tract, e.g., the renal pelvis, particularly over an extended period, preferably continuously and while the patient is ambulatory. US 2016/287369 A1 describes devices and methods for local delivery of drug to the upper urinary tract. US 2004/215169 A1 describes a drug-loaded medical device. WO 2017/132372 A1 describes multi-lumen drug delivery devices. US 2017/165460 A1 describes intravesical drug delivery devices and methods including elastic polymer-drug matrix systems.

### Brief Summary

The invention is defined in independent claim 1. Further aspects are defined in dependent claims 2-15. The methods of drug delivery or of use of a drug delivery device in the body of a patient described herein are not part of the claimed invention as they consist in non-patentable matter but are left for illustrative purposes.

Improved drug delivery devices and systems, and methods of drug delivery are provided herein. The drug delivery devices may be deployed directly into the renal pelvis via the natural lumens of the patient's body, i.e., via the ureter, bladder, and urethra, and the drug delivery devices can be wholly retained therein for delivery of drug over an extended period, e.g., several days or weeks, without relying on an external supply of drug and a catheter extending into the body during the period of administration. In some embodiments, the drug delivery devices are configured for insertion into the renal pelvis and sustained drug delivery therein, preferably providing a zero order release rate of therapeutically effective amounts of drug.

In one aspect, drug delivery devices configured for deployment in a renal pelvis of a patient are provided. In embodiments, the device includes (i) an elongated elastic body, wherein the elastic body has a guidewire lumen and a separate drug reservoir lumen; and (ii) a drug payload disposed in the drug reservoir lumen, wherein the drug payload includes a drug, wherein the drug delivery device is elastically deformable between (a) a deployment shape configured to pass the drug delivery device through a ureter and into the renal pelvis of the patient, and (b) a retention shape configured to mitigate migration of the device from the renal pelvis. In some preferred embodiments, the retention shape is helical. In some embodiments, the elastic body comprises (i) an outer tube comprising an elongated outer wall, and (ii) an elongated arcuate inner wall located within the outer tube and integrally connected to an inner surface the outer wall along two opposed edges of the arcuate inner wall, the outer and inner walls together defining (a) a guidewire lumen on a concave side of the inner wall, and (b) a drug reservoir lumen on an opposed convex side of the inner wall, the drug reservoir lumen being closed off at its opposed ends.

In another aspect, systems for local administration of a drug to the renal pelvis of a patient are provided. In some embodiments, the systems include a drug delivery device as described herein, and a guidewire deployment system for deploying the drug delivery device in the renal pelvis, the guidewire deployment system including (i) a guidewire, and (ii) a plunger device for pushing the drug delivery device over the guidewire.

In yet another aspect, methods (not claimed) are provided for administering a drug to a patient in need thereof. In some embodiments, the method includes deploying a drug delivery device into a renal pelvis of the patient; and continuously releasing drug from the deployed drug delivery device into urine in the renal pelvis over an extended treatment period of at least 24 hours, wherein the drug delivery device is wholly contained within the renal pelvis, with the optional exception of a retrieval string extending at least into the patient's ureter.

**Brief Description of the Drawings**

The detailed description is set forth with reference to the accompanying drawings. The use of the same reference numerals may indicate similar or identical items. Various embodiments may utilize elements and/or components other than those illustrated in the drawings, and some elements and/or components may not be present in various embodiments. Elements and/or components in the figures are not necessarily drawn to scale. Throughout this disclosure, depending on the context, singular and plural terminology may be used interchangeably.

**FIG. 1A** is a perspective view of one embodiment of a drug delivery device having a helical retention shape, as disclosed herein.

**FIG. 1B** is a perspective view of the drug delivery device of **FIG. 1A** in a straightened deployment shape.

**FIG. 1C** is a cross-sectional view of the drug delivery device shown in **FIG. 1B,** taken along cut-line C-C in **FIG. 1B.**

**FIG. 2** is a perspective view of one embodiment of a drug delivery device having a single coil retention shape, as disclosed herein.

**FIG. 3** is a cross-sectional view of an embodiment of a drug delivery device having a triple lumen design as disclosed herein.

**FIG. 4A** is a top cross-sectional view of one embodiment of a drug delivery device as disclosed herein, in a straightened configuration, with the cross-sectional plane extending longitudinally through the ends of the device and the drug reservoir lumen.

**FIG. 4B** is top plan view of the drug delivery device shown in **FIG. 4A.**

**FIG. 4C** is a side cross-sectional view of the drug delivery device shown in **FIG. 4A,** with the cross-sectional plane extending longitudinally through the ends of the device and through the drug reservoir lumen and the guidewire lumen.

**FIG. 5A** is a top cross-sectional view of another embodiment of a drug delivery device as disclosed herein, in a straightened configuration, with the cross-sectional plane extending longitudinally through the ends of the device and the drug reservoir lumen.

**FIG. 5B** is top plan view of the drug delivery device shown in **FIG. 5A.**

**FIG. 5C** is a side cross-sectional view of the drug delivery device shown in **FIG. 5A,** with the cross-sectional plane extending longitudinally through the ends of the device and through the drug reservoir lumen and the guidewire lumen.

**FIG. 6** is a perspective view of an embodiment of a drug delivery device having a helical retention shape and straight ends.

**FIG. 7** is a perspective view of another embodiment of a drug delivery device having a helical retention shape and straight ends.

**FIG. 8** is a perspective view of one embodiment of a drug delivery device having a helical retention shape with two relatively straight intermediate portions.

**FIG. 9** is a perspective view of one embodiment of a drug delivery device having a helical retention shape with a single relatively straight intermediate portion.

**FIG. 10A** is a perspective view showing a portion of one embodiment of a drug delivery device having a tapered end.

**FIG. 10B** is a side cross-sectional view of another embodiment of a drug delivery device having a tapered end, in a straightened configuration, with the cross-sectional plane extending longitudinally through the ends of the device and through the drug reservoir lumen and the guidewire lumen.

**FIG. 11** is a graph of in vitro drug release rates from drug delivery devices according to two different embodiments described herein.

**FIG. 12** is a graph of in vitro drug release rates from drug delivery devices according to two different embodiments described herein.

**FIG. 13** depicts a perspective view and a front view of one embodiment of a crescent shaped drug tablet as disclosed herein.

**FIG. 14A** is a perspective view of one embodiment of a deployment system for deployment of a drug delivery device, for example into the renal pelvis of a patient, as disclosed herein.

**FIG. 14B** is a perspective view of the deployment system shown in **FIG. 14A** after the advancement of a drug delivery device along a guidewire.

**FIG. 15** is a side cross-sectional view of one embodiment of a drug delivery device, in a straightened configuration, with the cross-sectional plane extending longitudinally through the ends of the device and through the drug reservoir lumen and the guidewire lumen, which depicts one embodiment of a connected retrieval string.

**FIG. 16** is a side cross-sectional view of one embodiment of a drug delivery device, in a straightened configuration, with the cross-sectional plane extending longitudinally through the ends of the device and through the drug reservoir lumen and the guidewire lumen, which depicts another embodiment of a connected retrieval string.

**FIG. 17** is a top cross-sectional view of a spacer for insertion into an end of a lumen of one embodiment of a drug delivery device as described herein, wherein the spacer includes a lateral through-hole through which a retrieval string is secured.

**FIG. 18** is a block diagram of an embodiment of a method for deploying a drug delivery device as described herein into the body of a patient, e.g., into the renal pelvis of the patient.

**FIG. 19** depicts an embodiment of a drug delivery device as described herein in a helical retention shape and located in a deployed position in the renal pelvis with an attached retrieval string extending from the drug delivery device through a ureter and into the bladder.

**Detailed Description**

Drug delivery devices, systems, and methods are provided herein that may be used to treat one or more conditions of the upper urinary tract. The one or more conditions of the upper urinary tract may include kidney cancer, ureter cancer, or other diseases that affect the kidneys and/or ureter. The drug delivery devices described herein may be deployed in the upper urinary tract, for example, in the renal pelvis. The drug delivery devices may release drug in the upper urinary tract for an extended period.

Following deployment, the devices may locally release drug continuously into the renal pelvis for several days or weeks, advantageously without the use of or need for an external pump, ureteral stent, or transurethral catheter. That is, the entire drug payload and means for controlling drug release beneficially are self-contained in the renal pelvis.

In some embodiments, the drug delivery for deployment in a renal pelvis of a patient includes (i) an elongated elastic body, wherein the elastic body has a guidewire lumen and a separate drug reservoir lumen; and (ii) a drug payload disposed in the drug reservoir lumen, wherein the drug payload includes a drug, wherein the drug delivery device is elastically deformable between (a) a deployment shape configured to pass the drug delivery device through a ureter and into the renal pelvis of the patient, and (b) a retention shape configured to mitigate migration of the device from the renal pelvis. In some preferred embodiments, the retention shape is helical. In some embodiments, the helical retention shape has from two to ten turns. The device is biased in the retention shape in the absence of a guidewire inserted into the guidewire lumen, by the elastic body (i) being thermally shape set to have the retention shape, and/or (ii) further including a retention frame lumen and a retention frame disposed in the retention frame lumen, wherein the retention frame is an elastic wire configured to bias the drug delivery device into the retention shape.

In some embodiments, the drug delivery device includes a retrieval string attached to the elongated elastic body, typically at an end portion, wherein the retrieval string has a length sufficient for an end of the retrieval string to reside in the patient's bladder when the drug delivery device is deployed in the renal pelvis.

The drug payload may be in any suitable form; however, in some preferred embodiments, the drug payload is the form of a powder or a plurality of tablets. In some embodiments, the elongated elastic body comprises a water permeable wall configured to permit urine to diffuse into the drug reservoir lumen to contact the drug payload. In some embodiments, the elongated elastic body includes a drug permeable wall adjacent to the drug reservoir lumen, the drug permeable wall being configured to permit the drug, in solution, to diffuse out of the device. The elongated elastic body may further include a drug impermeable wall adjacent to the drug reservoir lumen, for example, to limit the area available for transwall diffusion of drug and thereby slow/prolong the release of the drug. In some embodiments, the elastic body is formed of one or more thermoplastic polyurethanes, e.g., aliphatic polyethers. For example, in some embodiments, the drug permeable wall comprises Tecophilic™ polyurethane and the drug impermeable wall comprises a Tecoflex™ polyurethane. The drug permeable wall may be in the form of a drug permeable stripe extending the length of the elastic body.

The opposed ends of the drug reservoir lumen typically are each sealed by an end spacer, while the guidewire lumen remains open at its ends to permit passage therethrough of a guidewire.

The drug delivery device may further include one or more middle spacers disposed in the drug reservoir lumen, e.g., at a position approximately midway between opposed ends of the elongated elastic body. The elastic body, the end spacers, and/or the middle spacers may include a radio-opaque filler material to enable visualization of the device in vivo, for example to facilitate placement within the renal pelvis.

In some embodiments, the drug reservoir lumen has a crescent cross-sectional shape, and the drug payload comprises a powder or a plurality of crescent shaped tablets.

In some embodiments, the elastic body of the drug delivery device includes (i) an outer tube comprising an elongated outer wall, and (ii) an arcuate, elongated inner wall located within the outer tube and integrally connected to an inner surface of the outer wall along two opposed edges of the arcuate inner wall, wherein the outer and inner walls together define (a) a guidewire lumen on a concave side of the inner wall, and (b) a drug reservoir lumen on an opposed convex side of the inner wall, the drug reservoir lumen being closed off at its opposed ends.

In some embodiments, the elastic body of the drug delivery device has at least one straight end portion. In some embodiments, each of the two opposing end portions is a straight end portion. In some embodiments, the elastic body includes one or more intermediate straight portions and/or one or more helical portions, which straight or helical portions are disposed between the opposing end portions of the elastic body.

In some embodiments, the outer tube of the drug delivery devices includes two different materials of construction, of which a first material is permeable to water but impermeable to the drug when the drug is in solution, and a second material which is permeable to water and permeable to the drug when the drug is in solution. The second material may be adjacent to the drug payload. As used herein, the phrase "impermeable to the drug" refers to materials that are substantially impermeable to the drug, such that essentially no drug is released via the material over a therapeutic release period. In some embodiments, the drug delivery device is operable *in vivo* to permit water to diffuse through the first material and the second material into a drug reservoir lumen, and solubilize the drug payload and to controllably release solubilized drug from the drug delivery device through the second material. In one example, the second material is a drug permeable wall formed of Tecophilic™ polyurethane, and the first material is a drug impermeable wall formed of Tecoflex™ polyurethane. In some embodiments, these walls are laterally connected to one another, such that each directly bounds, and together define, a drug reservoir in the device. In some cases, this means the walls together define an annular body.

In some alternative embodiments, the first material is both water impermeable and drug impermeable, and the second material is both water permeable and drug permeable.

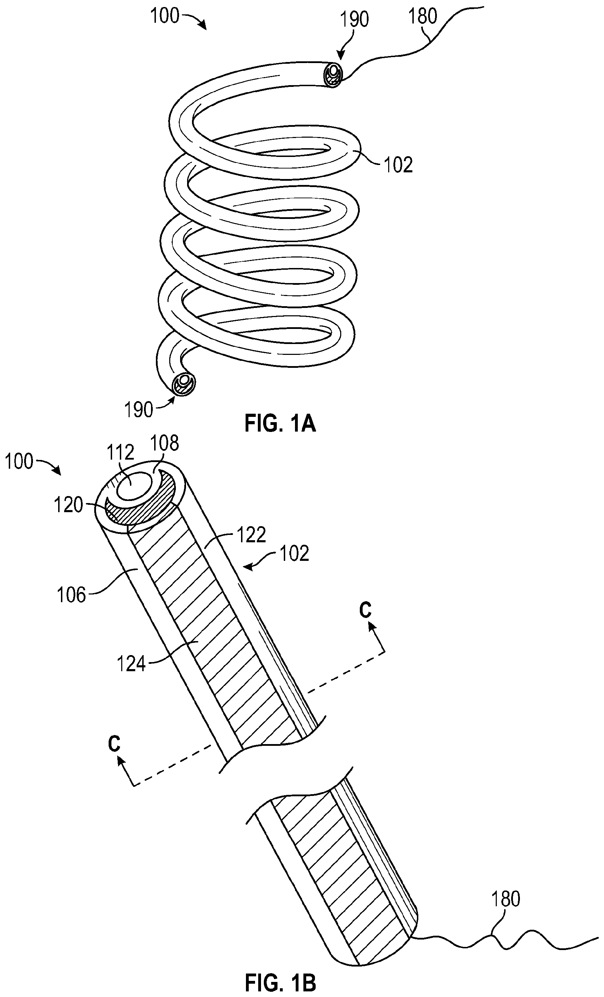

One embodiment of the drug delivery devices described herein is illustrated in **FIGS. 1A-1C. FIG. 1A** shows drug delivery device **100** which includes an elongated elastic body **102** and a retrieval string **180** attached to one end of the elongated elastic body **102.** In **FIG. 1A,** the device **100** is shown in a helical retention shape. **FIG. 1B** shows device **100** in a straightened deployment shape. As illustrated in **FIGS. 1B-1C,** elastic body **102** includes a guidewire lumen **112** extending therethrough and a separate drug reservoir lumen **114** extending within the elastic body **102.** A drug payload **116** is disposed in the crescent shaped drug reservoir lumen **114.** In this embodiment, the drug payload **116** is in the form of a plurality of crescent shaped tablets. The elastic body **102** includes an outer tube **104,** which comprises an elongated outer wall **106,** and an elongated, arcuate inner wall **108** located within the outer tube, and which is integrally connected to a surface of the outer wall along opposed edges of the arcuate inner wall **108.** The drug reservoir lumen **114** is defined longitudinally on a convex side of the inner wall **108,** and the guidewire lumen **112** is defined longitudinally on an opposed concave side of the inner wall **108.** The opposed ends of the drug reservoir lumen **114** are each sealed with an end spacer **120.**

In the embodiment illustrated in **FIGS. 1A-1C,** the elastic body is formed of two materials of construction, one material forming a drug impermeable wall **122** and the other material forming a drug permeable wall **124.** The drug permeable wall **124** defines at least a portion of the drug reservoir lumen **114,** and, therefore, is adjacent to the drug payload **116** that is disposed in the drug reservoir lumen **114.** The material forming the drug impermeable wall **122** may also form the arcuate inner wall **108** and the rest of the outer tube **104,** i.e., except for a stripe of the drug permeable material serving as the drug permeable wall **124,** as illustrated. In this embodiment, the ends **190** of the elastic body are blunt, i.e., untapered, and the end portions of the elastic body are helical when in the helical retention shape, as shown in **FIG. 1A.**

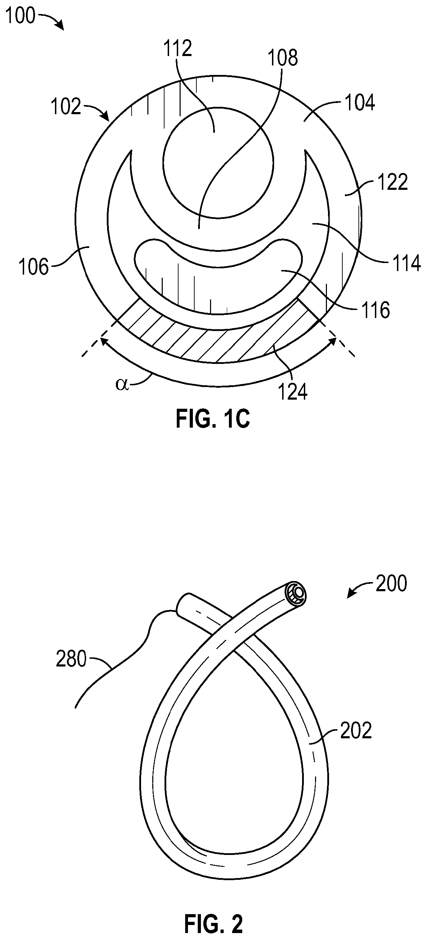

The drug permeable wall (stripe) **124** traversed the entire length of the drug delivery device **100** to provide a path for drug release. The width of the drug permeable stripe may be selected to adjust the effective rate of transwall diffusion of drug therethrough. Because the outer tube **104** has a circular cross-section, the width of the drug permeable stripe may be characterized by the arc angle of the stripe. In the embodiment illustrated in **FIG. 1C,** the arc angle, $\alpha$, is about 60 °. The angle and/or length and/or shape of the drug permeable stripe, however, can be varied to alter the drug release rate.

**FIG. 2** illustrates another embodiment of the drug delivery device. Here, the drug delivery device **200** which includes elastic body **202** and retrieval string **280.** It has a similar construction to that of drug delivery device **100,** but the retention shape is a single coil with overlapping end portions.

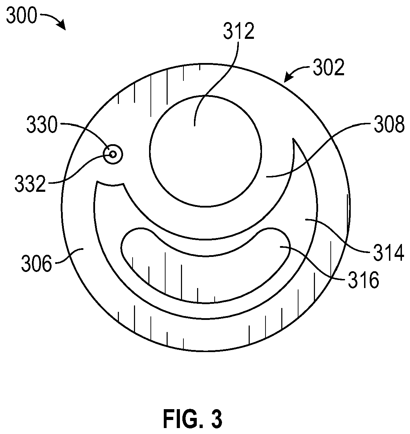

**FIG. 3** illustrates another embodiment of the drug delivery device. Here, the drug delivery device **300** includes elastic body **302.** Similar to the construction of drug delivery device **100,** it includes a guidewire lumen **312** extending therethrough and a separate, crescent shaped drug reservoir lumen **314** extending the length of the elastic body **302** and containing a drug payload **316** disposed in the drug reservoir lumen **314.** The elastic body **302** further includes a third lumen, which is a retention frame lumen **330,** in which a retention frame **332** is disposed. In this illustrated embodiment, the retention frame lumen **330** is defined near an intersection of an end edge of the arcuate inner wall **308** and the outer wall **306.** Similar to the construction of drug delivery device **100,** the outer wall **306** could be formed via a dual material polyurethane extrusion, and the drug reservoir lumen is defined at least partially by a stripe of a secondary polyurethane material that is permeable to water and a drug. In some cases, a majority of the tube is made from a water permeable polyurethane. The drug delivery device **300** may have a helical retention shape as at **FIG. 1A.**

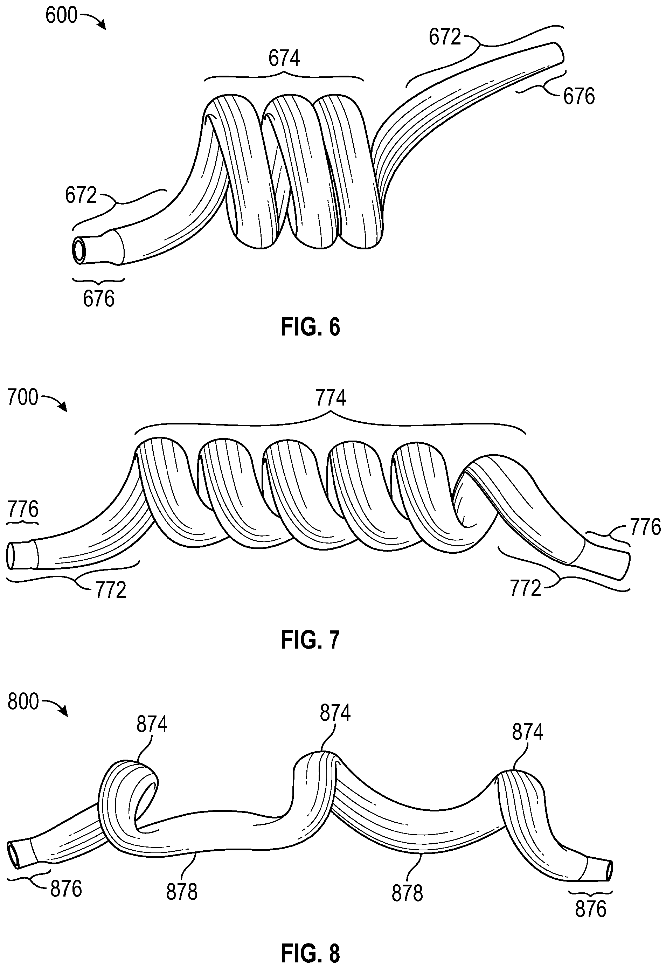

**FIG. 6** illustrates an embodiment of the drug delivery device **600** wherein the elastic body has a central helical portion **674** and opposing straight end portions **672.** The straight end portions **672** include tapered ends **676.** The straight end portions **672** extend in a direction approximately perpendicular to the planes of the turns of the central helical portion **674,** i.e., they extend in the longitudinal direction of the device. The straight end portions **672** may be thermally shape set. It is understood that even though these end portions are referred to as "straight" they need not literally conform to a single straight line. As evident in **FIG. 6,** the straight end portions **672** have a slight curvature, but are relatively straight when compared with the helical portion **674** and are distinct from the helical end portions of device **100** in **FIG. 1A.** The straight end portions **672** extend from opposite sides of the helical portion **674,** such that when the device is in the retentive shape, one straight end portion **672** (e.g., a leading end) is substantially aligned (diagonally across the device) with the other straight end portion **672** (e.g., a trailing end). This configuration advantageously (i) facilitates loading the drug delivery device onto a guidewire for deployment, (ii) may improve retention within the renal pelvis, and (iii) aids accurate placement of the device within the renal pelvis, because the leading straight end portion substantial maintains its position (because it does not coil) as it is pushed off the guidewire.

Another variation of this embodiment is shown in **FIG. 7.** The drug delivery device **700** likewise includes a central helical portion **774** and straight end portions **772.** However, the straight end portions do not align diagonally across the drug delivery device, but instead generally are disposed on the same side of the helical portion **774** and aligned along a line approximately parallel to the longitudinal axis of the drug delivery device **700.** The straight end portions **772** include tapered ends **776.**

In some other embodiments, the elongated body of the drug delivery device has one or more intermediate straight portions disposed between coils or between helices. Non-limiting examples of these embodiments are illustrated in **FIGS. 8-9.** These configurations may be particularly suited for use with patients having certain renal pelvis anatomical structures and/or sizes. For example, the intermediate straight portions may be suitably positioned within the pinch point areas of the renal pelvis of smaller kidneys, providing retention without undue pressure against the pinch point tissues. As with the straight end portions, the intermediate straight portions may have a slight curvature but are distinctly straighter than the turns or coils in the helical portions described above.

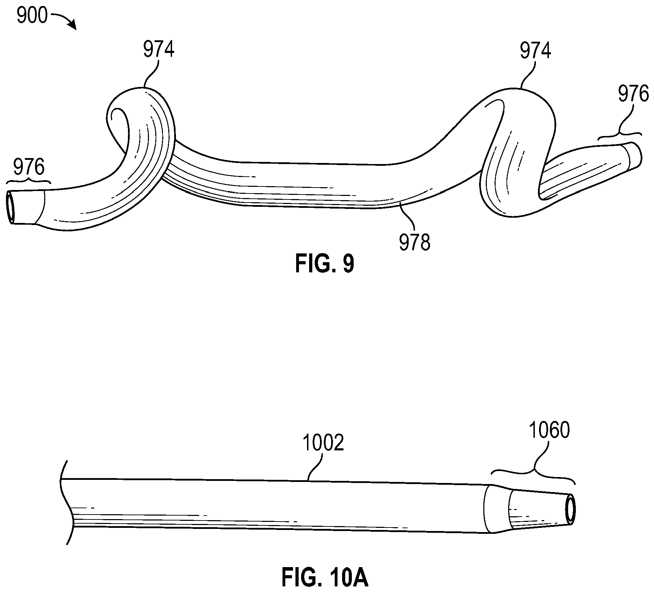

**FIG. 8** shows an embodiment of drug delivery device **800** in which the elongated body includes three loops **874** separated by two intermediate straight portions **878.** The device includes tapered ends **876. FIG. 9** shows an embodiment of drug delivery device **900** in which the elongated body includes two loops **974** separated by a single intermediate straight portions **978.** The device includes tapered ends **976.**

The size, number, and arrangement of intermediate straight portions and coil portions can be selected to accommodate both the various drug payload volumes needed and anatomical features of the renal pelvis for different patients. The overall size of the device and deployability also may impact the selected design of the drug delivery device. For example, the drug delivery device may have a shorter uncoiled length by using fewer turns in the helical portion. Alternatively, the outer coil diameter could be reduced resulting in tighter coils. A drug delivery device with an outer coil diameter of around 8 mm, or even as small as 7 mm, may be an effective retentive size for smaller kidneys, while a drug delivery device with an outer coil diameter of around 12 mm, or larger, may be an effective retentive size for larger kidneys. It is possible to decrease the outer coil diameter to 7 mm or smaller; however, it may become more challenging to load the device onto a guidewire and deploy it.

### Elastic Body

The elastic body serves as a housing for the drug payload and control release of the drug by transwall diffusion and/or osmotic pressure. In some embodiments, the elastic body includes walls that define the drug reservoir lumen, the guidewire lumen, and optionally a retention frame lumen. The walls forming the elastic body of the drug delivery devices described herein may be formed by an extrusion (or coextrusion) process such that the outer tube and inner walls are integrally connected. In some embodiments, the elastic body is thermally shape set to have a retention shape.

In some embodiments, the elastic body has an outer shape which is generally a long, thin tube, which in a retention shape is coiled upon itself, e.g., in a helical shape. The lumen extending the length of the elastic body may have essentially any size or shape that permits loading of a sufficient drug payload volume and can accommodate a guidewire of sufficient diameter to effect deployment of the device in the renal cavity. The walls bounding/defining the lumen must have a thickness for the device to be flexible yet suitably mechanically robust during deployment, use, and retrieval, while keeping the device small enough to fit within the renal pelvis and also providing that the wall thickness and area are dimensioned to achieve the desired water permeability and drug release kinetics.

The elastic body may be formed of any one or more materials having a flexibility sufficient to permit the drug delivery devices described herein to be deformable and, therefore, capable of assuming a deployment shape and a retention shape. In particular, the material may be a biocompatible elastomer, such as a thermoplastic elastomer, known in the art. The device typically is biased into the retention shape, such that in the absence of an applied load (e.g., a load effective to elastically deform toward/into a straightened shape or deployment shape), the device has the retention shape. The applied load may be imparted by the presence of a guidewire extending through a guidewire lumen in the elastic body.

In some embodiments, the outer tube of the drug delivery devices includes two different materials of construction, of which a first material is impermeable to the drug when the drug is in solution and a second material which is permeable to the drug when the drug is in solution. The second material may be adjacent to the drug payload. In other words, the second material is positioned so that at least a portion of drug on board a drug delivery device may contact at least a portion of the second material prior to, during, and/or after dissolution of the drug.

In some embodiments, the drug delivery devices described herein include a drug permeable stripe of the second material. The drug permeable stripe, in some embodiments, traverses about 50 % to 100 %, about 80 % to 100 %, about 90 % to 100 %, or 100 % of the longitudinal length of an outer tube. In some embodiments, the drug delivery devices described herein include an outer tube having two or more discrete portions formed from the second material. The one or more discrete portions may have any suitable size or dimensions.

When the drug delivery devices described herein include a drug permeable stripe of the second material, the drug permeable stripe may have an arc angle of about 30 ° to about 120 °, about 40 ° to about 120 °, about 45 ° to about 120 °, about 55 ° to about 120 °, about 60 ° to about 120 °, about 60 ° to about 110 °, about 60 ° to about 100 °, about 60 ° to about 90 °, or about 60 ° of the circumference of the outer tube in the cross section. The "arc angle" of a drug permeable strip, in other words, is the angle formed between a first line and a second line that extend from the center of an outer tube to the first side and the second side of a drug permeable stripe.

In some embodiments, an inner diameter of the outer tube is about 1.0 mm to about 2.3 mm. In some embodiments, an outer diameter of the outer tube is about 2.2 mm to about 2.4 mm. In some embodiments, a thickness of the walls (i.e., difference between the outer diameter and inner diameter) of an outer tube, first inner tube, and/or second inner tube is about 0.1 mm to about 1.4 mm, or about 0.15 mm to about 0.25 mm. In some embodiments, the thickness of the outer tube differs from the thickness of a first inner tube, a second inner tube, or a combination thereof.

Generally, the elastic body of the drug delivery devices described herein may be made of any one or more materials that impart the drug delivery devices with sufficient flexibility to permit the drug delivery device to be deformable between a retention shape and a deployment shape. When the elastic body include a first material and a second material, as described herein, the first material generally may include any flexible material, e.g., an elastomer, that is substantially impermeable to the drug contained in the drug delivery device. In some cases, this elastomer is water permeable. The second material generally may include any flexible material, e.g., an elastomer, that is permeable to water and the drug contained in the drug delivery device.

The first material and the second material may be selected from a variety of suitable materials, for example silicone, polyurethane, ethylene-vinyl acetate (EVA), thermoplastic silicone polyether polyurethane, aliphatic thermoplastic silicone polyether polyurethane, segmented polyether polyurethane, thermoplastic polyether polyurethane, thermoplastic polycarbonate polyurethane, BIONATE™ PCU, BIOSPAN™ SPU, CARBOSIL™ TSPCU, ELASTHANE™ TPU, PURSIL™ TSPU (DSM), other thermoplastic polyurethanes (TPUs), including aliphatic and aromatic, polycarbonate-based thermoplastic polyurethanes, such as CARBOTHANE™ TPU, TECOFLEX™ TPU, TECOTHANE™ TPU, PELLETHANE™ TPU, and TECOPHILIC™ TPU, and combinations or blends thereof.

In some embodiments, the second material is selected from TECOPHILIC™ thermoplastic polyurethane, HYDROTHANE™ thermoplastic polyurethane (AdvanSource Biomaterials Corp.), QUADRAPHILIC™ thermoplastic polyurethane (Biomerics, LLC) (ALC grades are aliphatic polycarbonate-based and ALE grades are aliphatic polyether-based hydrophilic polyurethanes), HYDROMED™ (AdvanSource Biomaterials Corp.), or DRYFLEX™ (HEXPOL TPE). Another hydrophilic polymer that may be selected for the second material is polyether block amide PEBAX® MV 1074 SA 01 MED (Arkema), which is a thermoplastic elastomer made of flexible and hydrophilic polyether and rigid polyamide.

In some embodiments, the elastic body includes one, two, or more water permeable thermoplastic polyurethanes. For example, the elastic body may be formed by an extrusion process, forming an elongated body that includes two parallel lumen and that consists of (i) more than 50 wt% of a first water permeable thermoplastic polyurethane, and (ii) less than 50 wt% of a second water permeable thermoplastic polyurethane. In some of these cases, the first water permeable thermoplastic polyurethane is drug impermeable, and the second water permeable thermoplastic polyurethane is drug permeable.

In some embodiments, the elastic body includes a first material and a second material, wherein the first material includes a tecoflex polyurethane, and the second material includes a tecophilic polyurethane. In some embodiments, the tecoflex polyurethane includes TECOFLEX™ EG-80A-B20, 20 % barium sulfate loaded tecoflex polyurethane (Lubrizol Life Sciences, USA). In some embodiments, the tecoflex polyurethane includes TECOFLEX™ EG-100A-B20, 20 % barium sulfate loaded tecoflex polyurethane (Lubrizol Life Sciences, USA). In some embodiments, the tecophilic polyurethane includes TECOPHILIC™ HP-60D-35 TPU (thermoplastic polyurethane) (Lubrizol Life Sciences, USA). In some embodiments for certain drugs, the tecoflex polyurethane is a drug impermeable material with a barium sulfate loading, which may allow for visualization during device insertion or retrieval. In some embodiments for certain drugs, the TECOPHILIC™ TPU is water permeable and drug permeable material that allows water into the drug delivery device, and also allows solubilized or liquid drug to traverse the second material and enter the space surrounding the drug delivery device.

For use in the renal pelvis, the deployed device should be compliant (i.e., easily flexed, soft feeling) in order to avoid or mitigate discomfort and irritation to the patient, but not too compliant that it easily, unintentionally migrates from the renal pelvis into a calyx or ureter, e.g., carried into the ureter with the flow of urine. Thus, the durometer of the first and second materials of construction may be important, and the proportion of a high durometer material may be limited in constructing an elastic body of a given size while keeping it suitably compliant in the renal pelvis. For example, TECOPHILIC™ thermoplastic polyurethane (Lubrizol Corp.) may have a Shore hardness greater than 70A, such as from 80A to 65D, while other drug impermeable thermoplastic polyurethanes may have a Shore hardness that is less than or greater than TECOPHILIC™, such as less than 90A. Accordingly, it can be advantageous to utilize a combination of two different polymeric materials, rather than making the device entirely of the water-swelling hydrophilic, drug-permeable second material, to achieve desired mechanical properties of the device.

In some embodiments, the drug delivery device may have tapered rather than blunted ends, such that the end portions of the elongated elastic body are narrower than the central portion of the elongated elastic body. **FIG. 10A** shows one embodiment of a drug delivery device in which elastic body **1002** includes a tapered end portion **1060.** The tapered end may advantageously increase the ease with which the drug delivery device can be inserted into a patient's ureter to reach the renal pelvis, as the taper reduces catching of tissue on the ends of the device. Similarly, the tapered end may also facilitate withdrawal of the device from the renal pelvis, easing entry into the ureter, and in some cases entry into the urethra, during the removal process. Accordingly either or both of the leading and trailing ends of the device may be tapered. The tapered end may be part of a straight end portion or a coiled end portion.

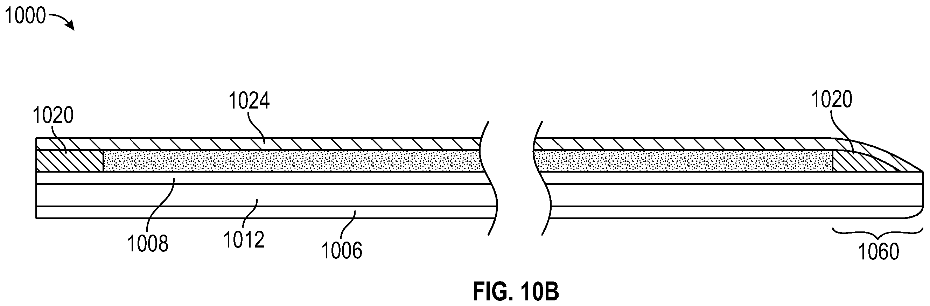

**FIG. 10B** shows a cross-section view of one embodiment of drug delivery device **1000** that has a tapered end portion **1060.** The elongated, elastic body includes outer wall **1006** with drug permeable stripe **1024.** The drug reservoir lumen is closed off by spacers **1020,** which may be installed therein prior to forming the end portion into a tapered end portion. As shown, the outer wall **1006,** drug permeable stripe **1024,** inner wall **1008,** and spacer **1020** may be plastically deformed (e.g., thinned, rounded) in the process of forming the taper toward the end of the drug delivery device; however, the process of forming the tapered end portion should not narrow or collapse the guidewire lumen **1012** at all or at least not to any degree that impedes passage of a guidewire therethrough. This may result in the tapered end portion being asymmetrical about a longitudinal central axis of the device, as shown in **FIG. 10B.**

The length of the elastic body of the drug delivery devices described herein may be selected depending upon a variety of factors including the amount of the drug payload required to be administered over the treatment in which the device is deployed, the release kinetics of the particular drug, and the overall shape and size needed to retain the device in the renal pelvis.

In some embodiments, the elastic body has a length of about 5 cm to about 15 cm, about 5 cm to about 12 cm, about 6 cm to about 12 cm, about 7 cm to about 12 cm, about 8 cm to about 12 cm, about 10 cm to about 12 cm, about 10 cm, or about 12 cm when in a relatively straightened deployment shape.

In some embodiments, the elastic body of the drug delivery devices described herein can be made to be completely or partially bioerodible so that no retrieval of the drug delivery device is required following release of drug. In some embodiments, the drug delivery device is partially bioerodible so that the drug delivery device, upon partial erosion, breaks into non-erodible pieces small enough to be excreted from the upper urinary tract. As used herein, the term "bioerodible" means that the drug delivery device, or part thereof, degrades *in vivo* by dissolution, enzymatic hydrolysis, erosion, resorption, or a combination thereof. In some embodiments, this degradation occurs at a time that does not interfere with the intended kinetics of drug release from the drug delivery device. For example, substantial erosion of the drug delivery device may not occur until after a drug is substantially or completely released. In some embodiments, the drug delivery device is erodible and the release of drug is controlled at least in part by the degradation or erosion characteristics of the erodible drug delivery device body.

### Drug Reservoir Lumen

The drug delivery devices described herein include a drug reservoir lumen. In some embodiments, the drug reservoir lumen is defined by and between (i) at least part of the elongated outer wall of the elastic body, and (ii) at least part of the arcuate, elongated inner wall of the elastic body. The outer and inner walls may be a single monolithic structure, e.g., an elastomeric structure, formed for example, by an extrusion process. The elongate drug reservoir lumen, in some embodiments, is closed off at its opposed ends.

The drug reservoir lumens of the drug delivery devices described herein generally may have any cross-sectional shape. In some embodiments, the drug reservoir lumens have a crescent cross-sectional shape. An example of a drug reservoir lumen having a crescent cross-sectional shape is depicted at **FIG. 1B** and **FIG. 1C.** Other cross-sectional shapes are envisioned, depending for example on the overall shape of the outer tube wall, the shape of the inner wall, and the presence, shape, and position of the guidewire lumen and the retention frame lumen, if any.

Each end of the drug reservoir lumen of the drug delivery devices described herein typically is sealed to contain the drug payload within the drug reservoir lumen. A drug reservoir lumen may be sealed thermally and/or with an adhesive (such as TECOFLEX® 1-MP TPU adhesive, Lubrizol, USA). The drug reservoir lumens may have a first end and a second end, and, in some embodiments, a first spacer and a second spacer are disposed in the first end and the second end of the drug reservoir lumen. The spacers may be formed of any biocompatible material. In some preferred embodiments, the spacers are heat set (melted/reformed) into a fixed sealing position in the drug reservoir lumen. In some other embodiments, the spacers may be retained in the drug reservoir lumen by friction, an adhesive, a mechanical feature (such as a tab or other locking feature), or a combination thereof. For examples, the spacers may have dimensions that exceed the cross-sectional dimensions of the drug reservoir lumen so that the spacers are retained by friction.

In some embodiments, the spacers include a radio-opaque filler material. In some embodiments, the material used to form the spacers is a barium-loaded material, which may permit the spacers to serve as points of visualization within the drug delivery device during and/or after deployment, removal, or a combination thereof.

## Guidewire Lumen

The drug delivery devices described herein may include a guidewire lumen. In some embodiments, the guidewire lumen is defined by and between (i) at least part of the elongated outer wall of the elastic body, and (ii) at least part of the arcuate, elongated inner wall of the elastic body. The guidewire lumen and the drug reservoir lumen are on opposite sides of the inner wall. The outer and inner walls may be a single monolithic structure, e.g., an elastomeric structure, formed for example, by an extrusion process. The elongate guidewire lumen has open ends to accommodate a guidewire extending through the guidewire lumen.

The guidewire lumen generally may have any cross-sectional size and shape that permits the drug delivery device to travel over a guidewire suited for renal pelvis deployment. In some embodiments, the guidewire lumen has a substantially circular cross-sectional shape. Other cross-sectional shapes are envisioned, however, including but not limited to cross-sectional shapes that are polygonal, e.g., hexagonal, octagonal, etc., or non-polygonal, e.g., elliptical, oval, etc.

## Retention and Deployment Shapes

The drug delivery devices described herein are elastically deformable between a relatively straightened shape suited for insertion through a lumen into the renal pelvis of a patient and a retention shape suited to retain the device within the renal pelvis. In some embodiments, the drug delivery device may naturally assume the retention shape and may be deformed, either manually or with the aid of an external apparatus, into the relatively straightened shape for insertion into the body. Once deployed the device may spontaneously or naturally return to the initial, retention shape for retention in the body, e.g., upon removal of straightening forces imparted by the presence of a guidewire extending through a guidewire lumen in the device body.

As used herein, the phrase "retention shape" generally denotes any shape suited for retaining the drug delivery devices described herein in the renal pelvis.

Similarly, the phrase "deployment shape" generally denotes any shape suited for deploying the drug delivery devices described herein into the renal pelvis through a patient's urethra, bladder, and ureter.

In some embodiments, the elastic body itself is configured to provide a retention shape function for the drug delivery device. That is, the elastic body may be formed of appropriate materials (e.g., a high durometer silicone) and dimensioned to impart the required elasticity and spring constant the drug delivery device requires. In some embodiments, the drug delivery devices include a retention frame to provide the retention shape function of the drug delivery device.

The elastic body in the retention shape may have essentially any shape and size that fits within the renal pelvis and that contacts the walls of the renal pelvis on more or sides, i.e., in multiple directions, thereby resisting migration and facilitating retention of the device. In addition, this shape can facilitate delivering drug to a number of different tissue surfaces within the kidney, which in the case of treating tumors, can advantageously make the drug delivery ambivalent to the location of the tumor. Examples of such shapes include coils, helices, and other configuration in which an elongated narrow tube can be wrapped back and forth, around itself, or the like, forming an overall 3-D shape with substantially round features, e.g., spherical, cylindrical. In these embodiments, the device can be elastically to an essentially linear shape for deployment over/through a guidewire or other deployment instrument.

The outer tube dimensions should be chosen to prevent kinking if/when the tube is thermally shaped. The critical bending radius of curvature (R*) of elastic tubes under pure bending condition can be approximated using the following equation:

$$R^* \approx \frac{3}{\sqrt{2}} \frac{r^2 \sqrt{1-v^2}}{w}$$

where v is Poisson's ratio, $r$ is the mean radius (i.e. (ID+OD)/4), and w is the tube wall thickness (Guarracino, F. 2003. On the analysis of cylindrical tubes under flexure: theoretical formulations, experimental data and finite element analyses. Thin Wall Struct; 41:127-147).

In some embodiments, the retention shape includes a coil. The term "coil", as used herein, generally refers to a single loop formed with an elastic body of a drug delivery device. An embodiment of a drug delivery device having a coil retention shape is depicted at **FIG. 2.**

In some embodiments, the retention shape includes a helix. The term "helix", as used herein, generally refers to an elastic body that forms two or more coils having the same or different coil diameters, wherein at least a portion of the elastic body resembles a corkscrew or helical spring. In some embodiments, the drug delivery devices having a helical retention shape include 2 turns, 3 turns, 4 turns, 5 turns, 6 turns, 7 turns, 8 turns, 9 turns, or 10 turns. Each turn (coil) of a drug delivery device may or may not contact an adjacent coil. Each coil of a drug delivery device may define shapes that are the same or different.

When the drug delivery devices described herein are in a retention shape, the drug delivery devices for an adult patient may have dimensions selected to retain the drug delivery device within the renal pelvis. For example, when the drug delivery device has a helical retention shape, the drug delivery device may have a length of about 8 cm to about 15 cm, about 8 cm to about 12 cm, or about 10 cm to about 12 cm when in a relatively straightened deployment shape. When in a helical retention shape, the drug delivery devices described herein may have a length of about 0.8 cm to about 2 cm, about 0.8 cm to about 1.8 cm, about 0.8 cm to about 1.5 cm, about 0.8 cm to about 1.4 cm, about 0.8 cm to about 1.2 cm, or about 1 cm. When in a helical retention shape, the "length" of the drug delivery device is measured along the axis that is encircled by the two or more coils. When in a helical retention shape, the drug delivery devices described herein may have a largest width of about 0.5 cm to about 1.4 cm, or about 0.5 cm to about 1 cm. When the two or more coils are substantially circular and have substantially the same coil diameters, the width the drug delivery device may be substantially identical regardless of the axis along which the width is measured (the axis corresponding to the width being perpendicular to the axis along which the length is measured). When the two or more coils are non-circular and/or have different coil diameters, the width may vary depending upon the axis along which it is measured. Therefore, the foregoing ranges define the "largest width" for some embodiments of the drug delivery devices described herein.

As a further example, when the drug delivery device has a coil retention shape, the length of the drug delivery device may be about 4 cm to about 8 cm, or about 4 cm to about 6 cm, when in a relatively straightened deployment shape. The drug delivery devices having a coil retention shape, may have a largest width of about 1 cm to about 3 cm, or about 1.5 cm to about 3 cm, when in the retention shape. Again, the elastic body may form a loop that is not substantially circular when the drug delivery device has a coil retention shape; therefore, the "largest width" is provided by the foregoing ranges.

For pediatric patients, the dimensions of the drug delivery devices may be smaller, e.g., proportional, for example, based on the anatomical size differences and/or on the drug dosage differences between the adult and pediatric patients. In addition to permitting insertion, the relatively small size of the drug delivery devices may also reduce patient discomfort and trauma to the upper urinary tract, including the renal pelvis. The drug delivery devices also may be small enough in the retention shape to permit slight mobility within the renal pelvis; however, the mobility, if any, preferably is minimized to prevent an end of the device from becoming entrained in urine and flowing into a ureter. Movement of the drug delivery devices may facilitate uniform drug delivery throughout the entire renal pelvis.

### Retention Frame

In some embodiments, the drug delivery devices may include a further elongated lumen that is separate from the guidewire lumen and the drug reservoir lumen. This further lumen may be a retention frame lumen in which a retention frame is disposed. The retention frame lumen may be closed at its ends.

The retention frame lumen includes an elastically deformable retention frame disposed therein. The retention frame serves to bias the elastic body of the drug delivery device into a retention shape. The retention frame may be an elastic wire. It may be formed of any elastic material effective to impart a suitable modulus or spring constant to the elastic body, and thus to the drug delivery device. In some embodiments, the retention frame is a wire formed from a superelastic alloy, such as nitinol or another superelastic alloy. For example, the elastic wire may be thermally shape set to have a coiled or helical retention shape.

### Drug Delivery Devices and Deployment Systems

In one aspect, a drug delivery system, or kit, is provided. In some embodiments, the drug delivery system includes a drug delivery device described herein and a deployment system for deploying the drug delivery device in the renal pelvis of a patient.

In some embodiments, the deployment system is a guidewire deployment system that includes (i) a guidewire, and (ii) a plunger device for pushing the drug delivery device over the guidewire. The guidewire may have a cross-sectional area that is sized and shaped for fitting through the guidewire lumen of the drug delivery device.

In some embodiments, the plunger device includes a plunger, a handle, a sheath extending between the plunger and the handle, the sheath transferring to the plunger a driving force applied to the handle, and an internal bore for receiving the guidewire, such that the plunger device can travel over the guidewire.

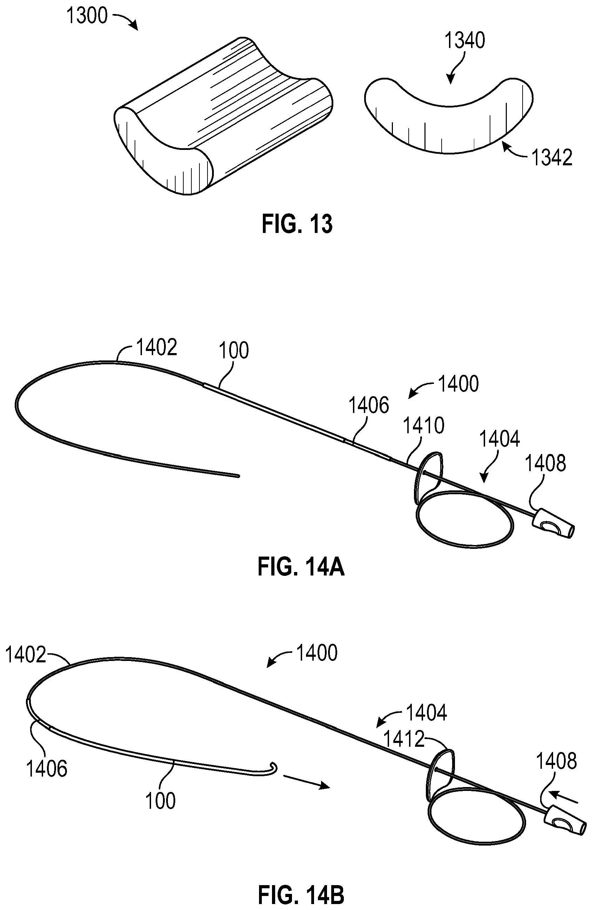

In some embodiments, the drug delivery device **100** of **FIGS. 1A-1C** may be deployed into the renal pelvis using a specialized guidewire deployment system. **FIGS. 14A-14B** depict an embodiment of such a guidewire deployment system **1400** for deploying a drug delivery device into the renal pelvis. The guidewire deployment system **1400** generally includes a guidewire **1402** and a plunger device **1404**. The guidewire **1402** is longer than the distance from the renal pelvis to the end of the urethra, such that when the guidewire **1402** is positioned in the ureter, its proximal end extends out from the urethra when its distal end is in the renal pelvis. The guidewire **1402** is sized and shaped for fitting through the guidewire lumen of the drug delivery device **100**. For example, the guidewire **1402** may have a cross-sectional area or diameter that is less than the cross-sectional area or diameter of the guidewire lumen.

The plunger device **1404** includes a plunger **1406** operatively connected to a handle **1408** by way of a sheath **1410**. The sheath **1410** is sufficiently rigid to transfer driving force from the handle **1408** to the plunger **1406**. The plunger **1406** and the sheath **1410** have an internal bore suited for threading the plunger device **1404** over the guidewire **1402**. Once the plunger device **1404** is so threaded, the handle **1408** is positioned on a proximal end of the guidewire **1402**, the plunger **1406** is positioned on the guidewire **1402** rearward of its distal end, and the rigid sheath **1410** extends from the handle **1408** to the plunger **1406** so that a driving force applied to the handle **1408** can be transferred to the plunger **1406**.

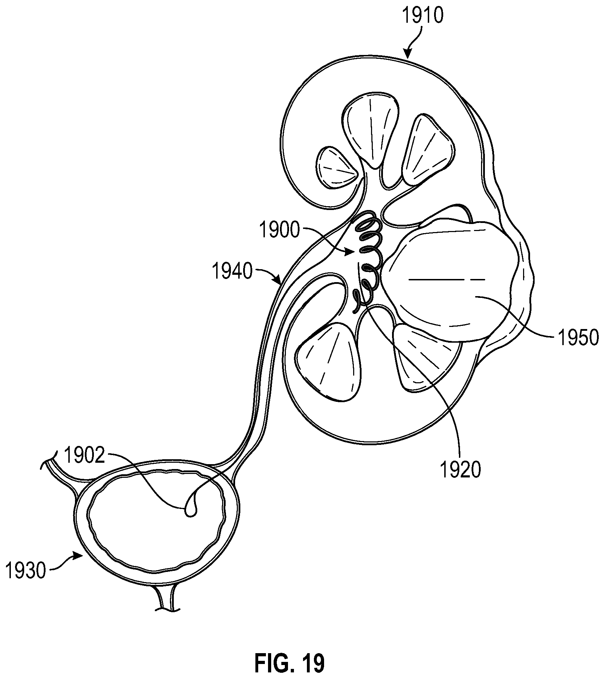

In use, the guidewire **1402** may be positioned in the ureter, such that its distal end is located in the renal pelvis and its proximal end is exposed outside of the patient's body. The drug delivery device **100** may be threaded onto the guidewire **1402**, with the guidewire **1402** passing through the guidewire lumen **112**, as depicted at **FIG. 1A** and **FIG. 1B**. The plunger device **1404** may be threaded onto the guidewire **1402** and the handle **1408** may be advanced, so that the rigid sheath **1410** travels along the guidewire **1402** to advance the plunger **1406** until the drug delivery device **100** is pushed from the guidewire **1402**. The guidewire **1402** then may be removed from the ureter, the bladder, and the urethra, leaving the drug delivery device in the renal pelvis, as illustrated in **FIG. 19**.

In some embodiments, the plunger device **1404** further includes a stop **1412**. The stop **1412** is positioned at an appropriate position on the plunger device **1404** so that the stop **1412** contacts the body of the patient once the drug delivery device **100** has separated from the guidewire **1402**. Thus, a user is notified that the drug delivery device **100** has been deployed *in vivo* so that the user can cease advancing the handle **1408** forward. In some embodiments, the stop **1412** may be adjustable to account for differences in anatomical sizes. The user may adjust the stop **1412** in advance of advancing the plunger device **1404**, as needed.

The guidewire **1402** may facilitate straightening the drug delivery device **100** from the retention shape, which is suited for retaining the drug delivery device **100** in the renal pelvis, into the deployment shape, which is suited for passing the drug delivery device **100** through the urethra and ureter. In some embodiments, the guidewire **1402** may be used in association with a separate deployment catheter. In such embodiments, the guidewire **1402** may be threaded through the deployment catheter, although other configurations are possible. The guidewire **1402** may also have a J-shape or a curved shape suited for deploying a drug delivery device through the male urethra. The guidewire deployment system **1400** and drug delivery device **100** also can be provided together in a package.

In some embodiments, any of the deployment instruments described herein with reference to **FIGS. 14A-14B** may be provided as part of a kit. The kit may include a package that houses one of the instruments and one of the drug delivery device described herein. For example, the kit may include a guidewire, a deployment catheter, a plunger device, and a drug delivery device configured for renal pelvis deployment and retention. The package may protect the packaged components before the deployment procedure. For example, the components may be sterilized together, and transported and stored together, in the package until needed for use. In such embodiments, the drug delivery devices described herein can be pre-loaded into or onto the deployment instrument before the deployment instrument is placed in the package, which eliminates the need to load the drug delivery device during the deployment procedure, reducing the number of steps in the procedure and reducing the risk that the drug delivery device will be dropped inadvertently or damaged during loading. However, the drug delivery device need not be pre-loaded. Instead, the drug delivery device can be provided with the deployment instrument or can be packaged separately. Also, a stylet can be either be packaged with the deployment instrument, packaged separately, or omitted completely. Regardless of which components are packaged together, the package may be sterilized, such as using gamma irradiation or ethylene oxide sterilization.

**FIG. 19** depicts an embodiment of a drug delivery device **1900** as described herein in a helical retention shape and located in a deployed position in the renal pelvis **1920** of the kidney **1910** with an attached retrieval string **1902** extending from the drug delivery device through a ureter **1940** and into the bladder **1930**. In this embodiment, the drug delivery device **1900** provides local drug delivery to a tumor **1950**, e.g., renal cell carcinoma. However, in other cases, the drug delivery device can be used to treat other diseases and disorders involving the kidneys besides cancer.

**Drug Payload and Drug**

Generally, the drug delivery devices provided herein include a drug payload disposed in a drug reservoir lumen. The drug payload includes at least one drug. In some embodiments, the drug payload is in a solid or semi-solid form. For example, the drug payload may be in a particulate form (e.g., a powder, granules), in the form of one more solid drug units (e.g., beads, tablets, capsules), or a combination thereof. The solid units typically are formed outside of elastic body to have a selectively imparted, uniform size and shape, and then the solid units are loaded into the drug reservoir lumen. In particular embodiments, the units should have a longest dimension configured to retain the orientation of the solid units (as loaded) within in the drug reservoir lumen while keeping that dimension short enough to have enough interstices (between the adjacent units) so that the loaded device can elastically deform along its length. In some embodiments, the drug payload includes a plurality of solid tablets, aligned end-to-end in the drug reservoir lumen.

The solid units may be made by a direct powder compaction or tableting process, a molding process, or other processes known in the pharmaceutical arts. In some embodiments, the crescent shaped tablets may be made by a modified tableting process, in which a powder mixture is loaded into a specially shaped mold and then compressed to bind the powder into a tablet. In some other embodiments, the drug payload may be loaded into a drug delivery device in workable form and then cured/solidified therein.

The drug payload may include a drug formulation, which includes at least one drug and at least one excipient. In some embodiments, the drug includes one or more active pharmaceutical ingredients (API), and the excipient includes one or more pharmaceutically acceptable excipients. The drug formulation can include essentially any therapeutic, prophylactic, or diagnostic agent, such as one that would be useful to deliver locally to the renal pelvis. The drug payload may consist only of the API, or one or more excipients may be included. As used herein, the term "drug" with reference to any specific drug described herein includes its alternative forms, such as salt forms, free acid forms, free base forms, and hydrates. The term "excipient" is known in the art, and representative examples of excipients useful in the present drug formulations may include ingredients such as binders, lubricants, glidants, disintegrants, colors, fillers, diluents, coatings, or preservatives, as well as other non-active ingredients to facilitate manufacturing, stability, dispersibility, wettability, and/or release kinetics of the drug or administering the drug formulation. The drug may be a small molecule, macromolecule, biologic, antimetabolite, or metabolite, among other forms/types of active ingredients. In some embodiments, the drug is a nucleoside analog.

In order to maximize the amount of drug that can be stored in and released from a given drug delivery device of a selected (small) size, the drug payload preferably includes a high weight fraction of drug or API, with a reduced or low weight fraction of excipients as are required for solid drug unit manufacturing and drug delivery device assembly and use considerations. For the purposes of this disclosure, terms such as "weight fraction," "weight percentage," and "percentage by weight" with reference to drug, or API, refers to the drug or API in the form employed, such as in salt form, free acid form, free base form, or hydrate form. For example, a solid drug unit that has 90% by weight of a drug in salt form may include less than 90% by weight of that drug in free base form.

In some embodiments, the drug payload is more than 50% by weight drug. In some embodiments, 75% or more of the weight of the drug payload is drug, with the remainder of the weight including excipients, such as lubricants and binders that facilitate making a solid drug unit. For the purposes of this disclosure, the term "high weight fraction" with reference to the drug or API means that excipients constitute less than 25 wt %, preferably less than 20 wt %, more preferably less than 15 wt %, and even more preferably less than 10 wt % of a drug payload. In some cases, the content includes about 75% or more of the weight of the drug payload. More particularly, the drug may include about 80% or more of the weight of the drug payload. For example, a drug may include between about 85% and about 99.9% of the weight of a solid drug unit. In some embodiments, an excipient content can be omitted completely.

In some embodiments, the drug and the excipient(s) are selected such that the solid drug form is water soluble, so that the solid drug form can be solubilized when the drug delivery device is located within the renal pelvis, to release the solubilized drug.

Individual solid drug units may have essentially any selected shape and dimension that fits within the drug reservoir lumen of the drug delivery devices described herein. In some embodiments, the solid drug units are sized and shaped such that the drug reservoir lumens are substantially filled by a selected number of solid drug units. Each solid drug unit may have a cross-sectional shape that substantially corresponds to a cross-sectional shape of the drug reservoir lumen of a particular housing. For example, the drug units may have an extruded crescent shape for positioning in a drug reservoir lumen having a crescent cross-sectional shape. Once loaded, the solid drug units can, in some embodiments, substantially fill the drug reservoir lumens.

In some embodiments, the solid drug units are shaped to align in a row within the drug reservoir lumen of the device. For example, each solid drug unit may have a cross-sectional shape that corresponds to the cross-sectional shape of the drug reservoir lumen, and each solid drug unit may have end face shapes that correspond to the end faces of adjacent solid drug units. The interstices or breaks between solid drug units can accommodate deformation or movement of the drug delivery device, such as during deployment, while permitting the individual drug units to retain their solid form.

Thus, the drug delivery devices may be relatively flexible or deformable despite being loaded with a solid drug, as each drug unit may be permitted to move with reference to adjacent drug units.

In some other embodiments, the drug payload includes a drug formulation that is in semi-solid form, such as an emulsion or suspension, a gel, or a paste. For example, the drug formulation may be a highly viscous emulsion or suspension. In some other embodiments, the drug formulation is in a liquid form.

The drug reservoir lumen may hold a number of drug tablets or other solid drug units. In some embodiments, the device holds 4 to 400 drug tablets, e.g., from 10 to 100 drug tablets, from 5 to 50 tablets, or from 10 to 40 tablets.

In some embodiments, the drug tablets have a cross-section that is crescent shaped. That is, the cross-section of the tablet may have a convex side and an opposed concave side which are connected at their ends/edges by shorter rounded sides. In other embodiments, not illustrated, the convex and concave sides may meet at a point, yielding a crescent shaped cross-section. For convenience, these kidney-shaped and crescent-shaped tablet cross-sectional areas will be referred to herein as "crescent" shaped. With a crescent shape, the drug tablet may take the approximate shape of a crescent shaped drug reservoir drug reservoir lumen, fitting into it and filling a substantial portion of the drug reservoir lumen's cross-sectional area, e.g., from 80% to 99% of it. The crescent-shaped tablets may have flat ends.

One example of a crescent-shaped tablet is shown in **FIG. 13.** The tablet **1300** has a crescent cross-sectional shape include a major curved surface **1342** and a minor curved surface **1340.** Although the major curved surface **1342** and minor curved surface **1340** are rounded curves, it is envisioned that these surfaces may be or include a planar surface (e.g., a major or minor curve in cross section could resemble three sides of a pentagon, three sides of a hexagon, etc.) The major curve and the minor curve may meet at a point at one or both ends of a tablet when viewed in cross-section, as when two overlapping circles form a crescent; or, the major curve and the minor curve may be connected at one or both ends of a table by a rounded portion of the table, as featured in the embodiment depicted at **FIG. 13.** A crescent-shaped tablet may have a structure in which the distance between the major curve and the minor curve is tapered, substantially constant, or a combination thereof. A tablet is considered to have a crescent-cross sectional shape if the greatest distance between its major curve and minor curve is no greater than 95% of the tablet's height.

The drug may be a low solubility drug. As used herein, the term "low solubility" refers to a drug having a solubility of about 0.01 mg/mL to about 10 mg/mL water at 37° C. In some other embodiments, the drug is a high solubility drug. As used herein, the term "high solubility" refers to a drug having a solubility above about 10 mg/mL water at 37° C. For example, the approximate solubilities of certain drug formulations are: trospium chloride: 500 mg/mL; lidocaine HCl: 680 mg/mL; lidocaine base: 8 mg/mL, gemcitabine HCl: 80 mg/mL; gemcitabine base: 15 mg/mL; oxybutynin HCl: 50 mg/mL; oxybutynin base: 0.012 mg/mL; and tolterodine tartrate: 12 mg/mL.

In some embodiments, the drug delivery devices are used to treat diseases of the upper urinary tract, including cancer of the upper urinary tract, such as upper urinary tract urothelial carcinoma. Drugs that may be used include antiproliferative agents, cytotoxic agents, chemotherapeutic agents, or combinations thereof. Representative examples of drugs which may be suitable for the treatment of upper urinary tract cancer include Bacillus Calmette Guerin (BCG) vaccine, docetaxel, cisplatin, doxorubicin, valrubicin, gemcitabine, mycobacterial cell wall-DNA complex (MCC), methotrexate, vinblastine, thiotepa, mitomycin (e.g., mitomycin C), fluorouracil, leuprolide, diethylstilbestrol, estramustine, megestrol acetate, cyproterone, flutamide, a selective estrogen receptor modulators (i.e. a SERM, such as tamoxifen), botulinum toxins, and cyclophosphamide. The drug may include a monoclonal antibody, a TNF inhibitor, an anti-leukin, or the like. The drug also may be an immunomodulator, such as a TLR agonist, including imiquimod or another TLR7 agonist. The drug also may be a kinase inhibitor, such as a fibroblast growth factor receptor-3 (FGFR3)-selective tyrosine kinase inhibitor, a phosphatidylinositol 3 kinase (PI3K) inhibitor, or a mitogen-activated protein kinase (MAPK) inhibitor, among others or combinations thereof. Other examples include celecoxib, erolotinib, gefitinib, paclitaxel, polyphenon E, valrubicin, neocarzinostatin, apaziquone, Belinostat, Ingenol mebutate, Urocidin (MCC), Proxinium (VB 4845), BC 819 (BioCancell Therapeutics), Keyhole limpet haemocyanin, LOR 2040 (Lorus Therapeutics), urocanic acid, OGX 427 (OncoGenex), and SCH 721015 (Schering-Plough). The drug treatment may be coupled with a conventional radiation or surgical therapy targeted to the cancerous tissue.

In some other embodiments, the drug delivery device may be used to treat pain, and the drug payload comprises an anesthetic agent, analgesic agent, and combinations thereof. For example, the anesthetic agent may be an aminoamide (e.g., lidocaine), an aminoester (e.g., benzocaine), or combinations thereof. In some embodiments, the analgesic agent includes an opioid agonist.

In some other embodiments, the drug delivery devices may be used to treat inflammatory conditions such as interstitial cystitis, radiation cystitis, painful bladder syndrome, prostatitis, urethritis, post-surgical pain, and kidney stones. Non-limiting examples of specific drugs for these conditions include lidocaine, glycosaminoglycans (e.g., chondroitin sulfate, sulodexide), pentosan polysulfate sodium (PPS), dimethyl sulfoxide (DMSO), oxybutynin, mitomycin C, heparin, flavoxate, ketorolac, cyclosporine, or combinations thereof. For kidney stones, the drug(s) may be selected to treat pain and/or to promote dissolution of renal stones.

In some embodiments, the drug delivery devices may be used to treat urinary incontinence, frequency, or urgency, including urge incontinence and neurogenic incontinence, as well as trigonitis. Drugs that may be used include anticholinergic agents, antispasmodic agents, anti-muscarinic agents, β-2 agonists, alpha adrenergics, anticonvulsants, norepinephrine uptake inhibitors, serotonin uptake inhibitors, calcium channel blockers, potassium channel openers, and muscle relaxants.

In some embodiments, the drug delivery devices may be used to treat infections involving the kidneys, ureters, bladder, and urethra. Antibiotics, antibacterial, antifungal, antiprotozoal, antiseptic, antiviral and other anti-infective agents can be administered for treatment of such infections.

In some embodiments, the drug is selected from lidocaine, gemcitabine, docetaxel, carboplatin, cisplatin, oxaliplatin, trospium, tolterodine, oxybutynin, and mitomycin C.

## Other Drug Delivery Device Features

### Imaging Features

The drug delivery devices described herein may include a radio-opaque portion or structure to facilitate detection or viewing (e.g., by X-ray imaging or fluoroscopy) of the drug delivery device by a medical practitioner as part of a deployment procedure and/or a retrieval procedure.

In some embodiments, the elastic body is constructed of a material that includes an imaging element. The imaging element may include a radio-opaque filler material, such as barium sulfate or another radio-opaque material known in the art. The imaging element may include one or more radiopaque marker bands fixed to the elastic body. Some elastic bodies may be made radio-opaque by blending radio-opaque fillers, such as barium sulfate or another suitable material, during the processing of a material from which the elastic body is formed. In some embodiments, the first material of which an elastic body is formed is a barium sulfate loaded material, *i.e.,* a material that includes barium sulfate. In some embodiments, at least one spacer of a drug delivery device includes an imaging material, including, but not limited to, a radio-opaque filler material, such as barium sulfate. In a preferred embodiment, the drug delivery device includes two end spacers and at least one intermediate spacer, all of which are disposed in the drug reservoir lumen. Ultrasound imaging or fluoroscopy may be used to image the drug delivery device *in vivo.* To improve visibility of a drug delivery device during insertion or otherwise, it is possible to increase the barium sulfate concentration within an elastic body (e.g., the first material, the second material, or a combination thereof), one or more spacers, or a combination thereof.

### Retrieval String

The drug delivery devices described herein may further include a retrieval feature, such as a retrieval string, a loop, a tab, or other structure that facilitates removal of the drug delivery device from the renal pelvis. In some embodiments, the retrieval feature is a retrieval string having a distal end fixed to an end of the drug delivery device and a proximal end that is configured to extend through a ureter and into the bladder following device deployment. Then, the device can be removed from the renal pelvis by grasping and pulling the proximal end of the retrieval string, e.g., following the end of the treatment period (e.g., following release of some or all of the drug payload into the renal pelvis).

In some embodiments, a deployed drug delivery device may be removed from the renal pelvis by engaging a retrieval string to pull the drug delivery device through the ureter, the bladder, and the urethra. The drug delivery device may be configured to assume a relatively narrow or linear shape when pulling the drug delivery device by the retrieval feature into the ureter and then into the urethra. The retrieval string has a first end portion that is attached to the drug delivery device, and an opposed second end portion that is engaged to pull the drug delivery device through the ureter, the bladder, and the urethra. The retrieval string may have a length sufficient for the second end of the retrieval string to reside in the bladder when a drug delivery device is deployed in the renal pelvis, as shown in **FIG. 19.** The retrieval string generally is formed of a biocompatible, woven or non-woven, material. The retrieval string, in some embodiments, is constructed of suture materials known in the art, e.g., silk, nylon, polyester, PVDF, and polypropylene. The retrieval string may be attached to any portion of a drug delivery device, including an end portion or a middle portion of the elongated body of the drug delivery device. In some embodiments, the drug delivery device two or more retrieval strings.

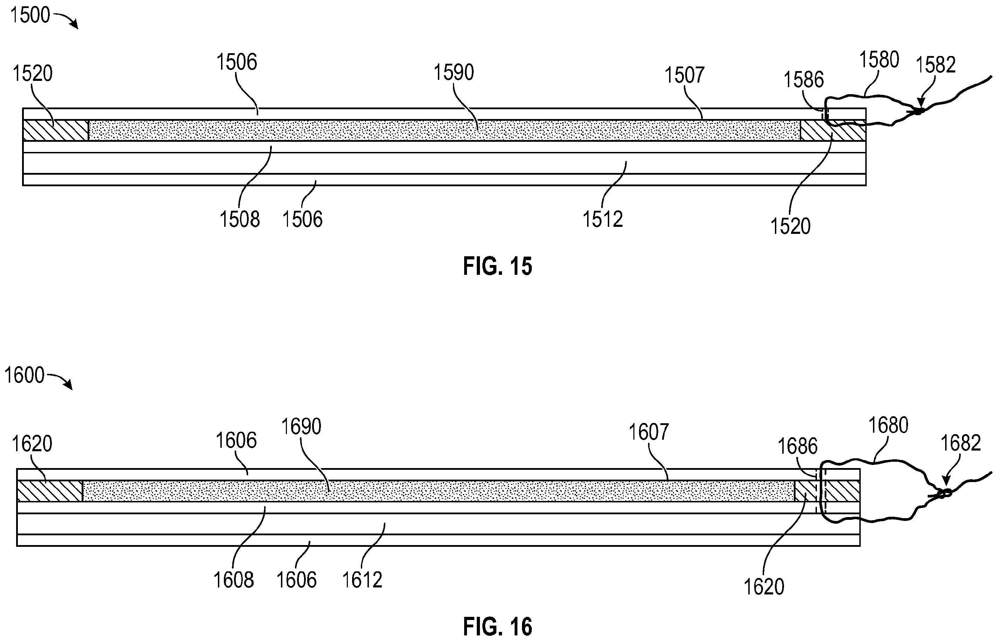

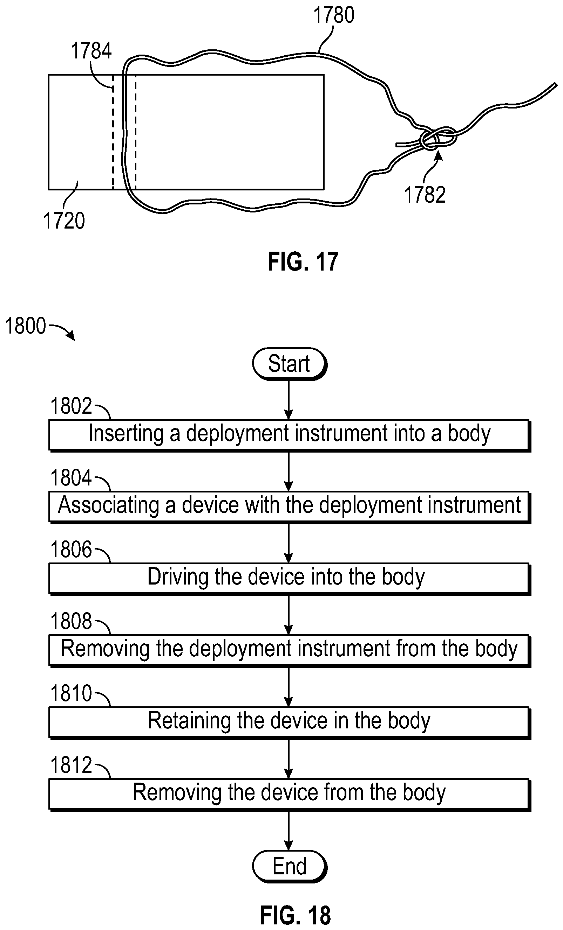

The retrieval string may be attached to the device body in a number of different ways, including different means and locations of connecting these components. For example, **FIGS. 15-16** show two different embodiments in which a retrieval string is secured to an end of a drug delivery device, and **FIG. 17** shows another embodiment of a retrieval string secured to an end spacer, before placement of the assembly within a lumen of a drug delivery device (not shown).

One embodiment is shown in **FIG. 15,** which illustrates a drug delivery device **1500** which has an elongated tubular body having outer wall **1506** and inner wall **1508** bounding (i) drug reservoir lumen **1507** which contains drug payload **1590,** and (ii) guidewire lumen **1512.** Spacers **1520** are secured within and closed off the ends of the drug reservoir lumen. The outer wall **1506** includes a hole **1586** through which a retrieval string **1580** passes. The retrieval string **1580** loops back on itself, with a first portion of the retrieval string being secured between a spacer **1520** and the outer wall **1506** and a second portion of the retrieval string passing along the outer surface of the outer wall, with the second portion of retrieval string being tied to the first portion of the retrieval strong at knot **1582.** Alternatively or in addition to the knot, the retrieval string **1580** may be held in place by either friction between the spacer **1520** and the inner portion of the outer wall **1506** and/or with aid of an adhesive. In a variation (not shown) of this embodiment, the hole extends into the spacer, and the retrieval string passes through both the hole in the outer wall and the hole in the spacer, such that the first portion of the retrieval string is secured between the spacer and the inner wall, instead of outer wall.

Another embodiment is shown in **FIG. 16,** which illustrates a drug delivery device **1600** which has an elongated tubular body having outer wall **1606** and inner wall **1608** bounding (i) drug reservoir lumen **1607** which contains drug payload **1690,** and (ii) guidewire lumen **1612.** Spacers **1620** are secured within and closed off the ends of the drug reservoir lumen. The outer wall **1606,** the inner wall **1608,** and one of the spacers **1620** together have a hole **1686** extending therethrough. The retrieval string **1580** passes through the hole **1686** and loops back on itself, with a first portion of the retrieval string passing along the outer surface of the outer wall and a second portion passing along the inner wall within the guidewire lumen, with the first portion of retrieval string being tied to the second portion of the retrieval strong at knot **1682.** Alternatively or in addition to the knot, the retrieval string **1680** may be held in place with aid of an adhesive. In one variation (not shown), the hole in the spacer is offset from the holes in the outer and inner walls (instead of being aligned with one another as in the illustrated embodiment), such that a portion of the retrieval string is trapped by frictional engagement between the spacer and the inner and/or outer walls. In another variation (not shown), the hole may extend laterally through the outer wall of the device body at two points without penetrating the inner wall.

One embodiment of a spacer with a retrieval string is shown in **FIG. 17.** The spacer **1720** is an elongated cylindrical body that has a hole **1784** extending therethrough in a direction normal to the longitudinal axis of the body. A retrieval string **1780** extends through the hole **1784** and loops back and is tied to itself at knot **1782.** This assembly of spacer and retrieval string may then be secured within an end of a drug reservoir lumen in a drug delivery device as described herein.

In yet another embodiment, the retrieval string is directly embedded into a spacer. For example, the retrieval string may be installed during formation of the spacer, e.g., included with a thermoplastic material during a molding step to make the spacer. In still another embodiment, the retrieval string is directly attached to the device body at a part and/or location not including the spacer. For example, the retrieval string may be tied and/or secured with an adhesive to a sidewall of the device body.

In some embodiments, the drug delivery device may include a retrieval tab. The retrieval tab may be part of the device body, e.g., integrally formed with it. The device may be removed from the renal pelvis by grasping, or otherwise engaging, the retrieval tab with forceps or another instrument to pull the drug delivery device through the ureter, bladder, and urethra. The distal end of the instrument may be guided into the renal pelvis through the urethra, bladder, and ureter to reach the device, for example, with a ureteroscope. The drug delivery device may be configured to assume a relatively narrow or linear shape when pulling the drug delivery device by the tab into the urethra or the ureter.

### Methods for Drug Delivery

The drug delivery devices, systems, and methods disclosed herein are particularly adapted for use in humans. It may also be adapted for use in other mammals such as in veterinary or livestock applications. Accordingly, the term "patient" may refer to a human or another mammalian subject.

In some embodiments, the methods of providing controlled release of drug to a patient include (i) deploying a drug delivery device as described herein into the patient, for example, into the renal pelvis; and (ii) releasing a drug from a drug reservoir lumen within the device, continuously over a sustained period, i.e., more than 24 hours, and into the local environment of the renal pelvis. For example, the drug may pass into urine with the renal pelvis and then diffuse into adjacent tissues. In some embodiments, the drug is released locally to tissues at the deployment site within the renal pelvis. In some embodiments, the released drug may partition to adjacent tissues. In some embodiments, the released drug may be carried in urine from the renal pelvis to treat the ureteral, the bladder, and urethra. Having the drug delivery device reside wholly in the renal pelvis without the presence of a ureteral stent may be a particularly advantageous way to treat tissues of the upper tract (e.g., kidney and ureter) continuously with a therapeutic agent over an extended period.

The release may, for example, occur via transwall diffusion through an elastic body of the device, or may occur by osmotic pressure driving the drug through one or more apertures in the body of the device. In some embodiments, the device has an elastic body that includes an outer tube that includes (i) a first material that is impermeable to the drug, and (ii) a second material that is permeable to the drug, and drug diffuses (by transwall diffusion) only through the second material. The drug delivery device may include any features, or combinations of features, described herein.

In some embodiments, urine diffuses into the drug reservoir lumen by transwall diffusion through the first material, the second material, or a combination thereof, and contacts the drug contained the drug reservoir lumen, producing a solution of the drug, which subsequently diffuses by transwall diffusion through the second material and into the renal pelvis.

In some embodiments, the deployed drug delivery device remains in the renal pelvis for a prescribed treatment period, controllably releasing drug over the prescribed treatment period. The drug delivery device may deliver drug for a treatment period of several days, weeks, months, or more following its deployment.

Once deployed, the drug delivery device releases a desired quantity of drug over a desired, predetermined period. In preferred embodiments, the drug delivery device can deliver the desired dose of drug continuously over an extended period of 24 hours or more, e.g., 1 to 90 days, 2 to 60 days, 3 to 45 days, 3 to 30 days, 3 to 21 days, 3 to 14 days, 7 to 45 days, 7 to 30 days, 7 to 14 days, 7 to 10 days, 3 to 10 days, 24 to 72 hours, 36 to 60 hours, or 48 to 90 hours. The rate of delivery and dosage of the drug can be selected depending upon the drug being delivered and the disease or condition being treated. In some embodiments, a rate of release of the drug from the drug delivery device is substantially zero order over at least 36 hours. In some embodiments, a rate of the release of the drug from the drug delivery device is substantially zero order over at least 7 days.

In some embodiments, the drug is gemcitabine, e.g., gemcitabine hydrochloride. In some of these embodiments, at least 25 mg/day of gemcitabine is released over 7 days. In another embodiment, at least 1 mg/day of gemcitabine hydrochloride is released over 7 days to 3 months.

In some embodiments, elution of drug from the drug delivery device occurs following dissolution of the drug within the drug delivery device. For example, urine enters the drug delivery device, contacts the drug and solubilizes the drug, and thereafter the dissolved drug is released from the drug delivery device, e.g., by diffusion and/or osmotic pumping. In some embodiments, releasing the drug from the drug delivery device includes solubilizing the drug with water imbibed through the second material of the outer tube, or both the first material and the second material of the outer tube.

In some embodiments, the drug delivery device may have two payloads of drug that are released at different times. The first payload may be adapted for relatively quick release, while the second payload may be adapted for more extended release. The drugs in the two payloads may be the same drug or two different drugs.

### *Methods of Device Deployment and Retrieval*

Generally, the methods include deploying a drug delivery device as described herein in the renal pelvis of a patient, wherein the drug delivery device is wholly contained in the renal pelvis, with the optional exception of a retrieval string configured to extend into the bladder, or through the bladder and urethra, of the patient. The drug delivery device may be retrieved from the body, such as in cases in which the drug delivery device is non-resorbable or otherwise needs to be removed. In an alternative embodiment, the drug delivery device is completely or partially bioerodible, resorbable, or biodegradable, such that retrieval is unnecessary, as either the entire drug delivery device is resorbed or the drug delivery device sufficiently degrades into small enough pieces for expulsion with urine flowing from the renal pelvis. In embodiments, the drug delivery device may not be retrieved or resorbed until some of the drug, or preferably most or all of the drug, has been released. If needed, a new drug-loaded drug delivery device may subsequently be deployed, during the same procedure as the retrieval or at a later time.

In some embodiments, deploying the drug delivery device in the patient includes inserting the drug delivery device into the renal pelvis of the patient via a deployment instrument. In some embodiments, deploying the drug delivery device in the renal pelvis of the patient includes elastically deforming the drug delivery device into the deployment shape; inserting the drug delivery device through the patient's urethra, bladder, and ureter; and releasing the drug delivery device into the patient's renal pelvis such that the drug delivery devices assumes the retention shape suited to prevent or mitigate migration of the drug delivery device from the renal pelvis, e.g., into the ureter or a calyx. In some embodiments, deploying the drug delivery device in the patent includes inserting a guidewire through the urethra, bladder, ureter, and into the renal pelvis of the patient; advancing the drug delivery device along the guidewire, the guidewire being positioned within the guidewire lumen, until the drug delivery device is positioned in the renal pelvis; and then retracting the guidewire from the patient and from the guidewire lumen. In some embodiments, the drug delivery device is elastically deformable between a coiled or helical retention shape and an relatively straightened insertion shape, and wherein during the step of advancing the drug delivery device along the guidewire, the guidewire located in the guidewire lumen exerts a load on the drug delivery device to bias the drug delivery device into the relatively straightened insertion shape, and following the step of retracting of the guidewire from the guidewire lumen, the drug delivery device elastically returns to the coiled or helical retention shape suited to retain the drug delivery device within the renal pelvis of a patient. In some embodiments, the drug delivery device includes a radiopaque marker or agent, and the deployment includes determining placement of the drug delivery device in the patient by radiography.

FIG. 18 is a block diagram illustrating an embodiment of a method 1800 of use of the drug delivery devices described herein in the body of a patient, e.g., the renal pelvis. In block 1802, a deployment instrument is inserted into the body, providing a route through the patient's urethra, bladder, and one of the ureters, to reach the renal pelvis. Inserting the deployment instrument generally includes inserting the deployment instrument into the urethra, bladder, and ureter, and driving the deployment instrument forward until a distal end is positioned in the renal pelvis, while a proximal end remains outside of the body. In embodiments, the drug delivery device is deployed into the renal pelvis of a patient in an independent procedure or in conjunction with another urological procedure (e.g., lithotripsy) or surgery, either before, during, or after the other procedure.

In some embodiments, the deployment instrument is inserted into the body in block **1802** in association with a cystoscope or ureterscope, which permits visualizing the deployment procedure. In some embodiments, inserting the deployment instrument into the body in block **1802** also includes verifying a distal end of the deployment instrument has become positioned in the renal pelvis. The location of the distal end can be verified by visualizing the distal end of the deployment instrument with a cystoscope, an ultrasound or x-ray. Also in some embodiments, inserting the deployment instrument into the body in block **1802** also includes securing a distal end of the deployment instrument in the renal pelvis, such as by inflating a balloon positioned on the distal end.

In block **1804,** the drug delivery device is operably associated with the deployment instrument. The type of association depends on the deployment instruments involved. If the instrument is a luminal one (e.g., cystoscope, ureterscope, catheter, or the like), then the association step include inserting the drug delivery device into a lumen of the instrument. If the instrument includes a guidewire, then the association step includes inserting the guidewire into a guidewire lumen of the drug delivery device. Combinations of different deployment instruments and operable associations may be used. In typical embodiments, the operable associate includes elastically deforming the drug delivery device from its retention shape into its deployment shape. A lubricant may be used to facilitate sliding engagement between the deployment instrument and the drug delivery device when they are operably associated with one another. In some embodiments, the drug delivery device is preloaded onto/into the deployment instrument before the deployment instrument is inserted into the body. In such cases, the order of blocks **1802** and **1804** is reversed.

In block **1806,** the drug delivery device is driven into the renal pelvis. In embodiments, this step includes pushing the drug delivery device toward the distal end of the deployment instrument until the drug delivery device is separated from the deployment instrument (with the optional exception of a retrieval string, the distal end of which may remain with the deployment instrument), releasing the drug delivery device into the renal pelvis. For a luminal deployment instrument, the drug delivery device is pushed out of an opening in the distal end portion of the instrument, for example, using a stylet and/or an incompressible fluid (e.g., water, lubricant) advanced into through the lumen behind the drug delivery device. For a guidewire type deployment instrument, the drug delivery device is pushed off of the distal end of the guidewire, for example using a plunger that also rides over the guidewire. In some embodiments, following separation of the drug delivery device from the deployment instrument, the drug delivery device elastically returns, typically spontaneously, to its retention shape, as the device is no longer under a straightening-inducing load from the deployment instrument. In some embodiments, driving the drug delivery device into the renal pelvis in block **1806** includes observing the drug delivery device in the renal pelvis to ensure the drug delivery device was properly deployed. For example, the drug delivery device may be observed using an ureterscope, ultrasound, or x-ray.

In block **1808,** the deployment instrument is removed from the patient's body by withdrawing the instrument from the renal pelvis, the ureter, the bladder, and then the urethra. If the deployment instrument includes multiple components, the components may be withdrawn simultaneously or serially depending upon the components and manner desired to minimize patient discomfort and/or trauma the luminal tissues through which the deployment instrument passes. If the drug delivery device includes an attached retrieval string, then the free, distal end of the retrieval string may be withdrawn with the deployment instrument and released at the desired location within the patient (e.g., within the bladder).

Thereafter, in block **1810,** the drug delivery device remains deployed in the renal pelvis for a selected treatment period, releasing drug from the drug payload at a selected rate/amount over the treatment period. That is, once deployed *in vivo,* the drug delivery device releases drug from its drug payload for the treatment (or prophylaxis) of one or more diseases or conditions.

In block **1812,** the drug delivery device is removed from the patient's body, for example upon completion of the treatment period, or release of all or a majority of the drug payload. This step may include inserting a removal instrument into the body, locating the drug delivery device, or a part thereof, and pulling the drug delivery device from the body with the aid of the removal instrument. The removal instrument may include a cystoscope, catheter, or ureteroscope, and may further include forceps, lariat, or another grasping instrument. In embodiments in which the drug delivery device includes a retrieval string, the method of removing the device from the patient may include grasping the distal end portion of the retrieval string, which may be located in the patient's bladder, and then removing the drug delivery device from the patient through the ureter, bladder, and urethra, by pulling the retrieval string. In some embodiments, the drug delivery device may be pulled into a distal end portion of a luminal removal instrument, causing the device to fold upon itself or assume the deployment shape as it enters the removal instrument. In some embodiments, forceps passed through the removal instrument may be used to grasp the drug delivery device and pull it partially or completely into the removal instrument before withdrawing the removal instrument. In some embodiments, the removal instrument may include a magnet for magnetically coupling to a part of the drug delivery device that is configured for this purpose. Magnetic coupling advantageously can be done in a blind procedure, i.e., it is not necessary to visualize the device to secure it with the removal instrument. In some other embodiments, after any part of the drug delivery device is securely grasped (e.g., by the retrieval string), the removal instrument is withdrawn from the patient's body, with the drug delivery device trailing behind, not within, the removal instrument. In those embodiments, the drug delivery device may be pulled into the deployment shape as it enters the ureter and urethra, and these tissue structures maintain the drug delivery device in its deployment shape while passing therethrough. In various procedures, the drug delivery device may be pulled through the removal instrument, and thereafter the removal instrument may be removed from the body, or alternatively, the drug delivery device and removal instrument may be removed from the body simultaneously.

The drug delivery device in method **1800** can be any suitable drug delivery device described herein, including the embodiments of the drug delivery device **100** depicted at **FIGS. 1A-1C,** and the deployment instrument **1400** may be the instrument depicted at **FIGS. 14A-14B.** In one example, the drug delivery device is deployed by guiding the drug delivery device with a deployment instrument, e.g., a ureteral catheter and/or guidewire system, and releasing the drug delivery device from the deployment instrument into the renal pelvis. In those cases, the drug delivery device can assume a retention shape (e.g., a coiled or helical shape) once the drug delivery device emerges from the deployment instrument into the renal pelvis. The deployment instrument may include a guidewire deployment system as described herein. In some embodiments, deploying the drug delivery device in the renal pelvis of the patient includes (i) elastically deforming the drug delivery device into the deployment shape; (ii) inserting the drug delivery device through the patient's urethra, bladder, and ureter; and (iii) releasing the drug delivery device into the patient's renal pelvis such that the drug delivery device assumes the retention shape suited to prevent unwanted migration of the device.

### Methods of Treatment

The deployed drug delivery device may release one or more drugs locally to the renal pelvis of a patient for local or regional treatment or prophylaxis of a wide variety of diseases or conditions. Non-limiting examples include urinary tract infections, kidney infections (pyelonephritis), renal cell carcinoma, hyperfibrinolysis, upper tract urothelial carcinoma, and urinary stones, such as kidney stones, ureteral stones, and bladder stones. Treatments of other diseases and conditions are also envisioned.

In one embodiment, the patient is in need of treatment and/or prophylaxis of stones. Non-limiting examples of the drug to be delivered by the device include antimicrobials, alkalinizing agents, acidification agents, urease inhibitors, anti-inflammatories, and antifibrotics. In some embodiments, the drug delivery device is inserted into the patient following treating the patient with extracorporeal shock wave lithotripsy (ESWL) for treatment of kidney stones in the patient. The migration of smaller stone fragments to the lower part of the kidney or the lower pole calyces has been observed to occur post-ESWL treatment due to gravity and anatomical configuration (Bourdoumis, et al., "Lower Pole Stone Management" Med Surg Urol S1:002 (2012)). These stone fragments become relocated in the lower calyces and act as a nucleus for new stone formation leading to lower pole calyceal lithiasis. Accordingly, in embodiments of the present method, a drug that inhibits stone formation is released from the deployed device and into the renal pelvis. The released drug may become more concentrated and effective in these lower calyces due to gravity and anatomical configuration.

In one embodiment, the patient is in need of treatment or prophylaxis of a urinary tract infection (UTI) or pyelonephritis. In a particular embodiment, the method of treatment or prophylaxis includes delivering via the drug delivery device an antimicrobial agent. The antimicrobial agent may be an antibiotic, antibacterial, antifungal, antiviral, antiparasitic, disinfectant, or antiseptic agent known in the art. In certain embodiments, the antimicrobial may an aminoglycoside, a penem, or an iron mimetic. Non-limiting examples of specific antimicrobial agents that may be used in the methods of treatment or prophylaxis of UTI or pyelonephritis include trimethoprim/sulfamethoxazole, trimethoprim, ciprofloxacin, levofloxacin, norfloxacin, gatifloxacin, ofloxacin, nitrofurantoin, fosfomycin, pivmecillinam, cefpodoxime proxetil, ceftibuten, cefotaxime, ceftriaxone, ceftazidime, cefepime, amoxicillin/clavulanic acid, piperacillin/tazobactam, gentamicin, amikacin, ertapenem, imipenem/cilastatin, meropenem, doripenem, aztreonam, a gallium-based iron mimetic, and combinations thereof. In another embodiment, the "drug" administered to the patient includes an attenuated bacteria/pathogen for colonizing the genitourinary tract with a non-pathogenic bacteria to prevent recurrent urinary tract infection or pyelonephritis.

In another embodiment, the patient is in need of treatment of renal cell carcinoma. In particular embodiments, the method of treatment includes delivering via the drug delivery device an anti-angiogenesis agent, a tyrosine kinase inhibitor, an mTOR inhibitor, or a combination thereof. Non-limiting examples of specific drugs that may be used in the methods of treatment for renal cell carcinoma include everolimus, aldesleukin, bevacizumab, axitinib, sorafenib tosylate, pazopanib hydrochloride, aldesleukin, sunitnib malate, temsirolimus, and combinations thereof. Other treatments may be used in conjunction with the use of the device drug delivery devices described herein. For example, the method of treatment may further include surgery, for example a partial nephrectomy; radiation; or systemic chemotherapy.

In still another embodiment, the patient is in need of treatment of upper tract urothelial carcinoma, or transition cell cancer of the renal pelvis and ureter. Non-limiting examples of specific drugs that may be used in the methods of treatment for upper tract urothelial carcinoma include Bacillus Calmette-Guerin (BCG), mitomycin C, BCG/interferon, interferon (IFN)-2a, epirubicin, thiotepa, doxorubicin, gemcitabine, and combinations thereof. Other treatments may be used in conjunction with the use of the drug delivery devices described herein. For example, the method of treatment may further include surgery; radiation; or systemic chemotherapy.

In yet another embodiment, the patient is in need of treatment of hyperfibrinolysis. Non-limiting examples of specific drugs that may be used in the methods of treatment for hyperfibrinolysis include tranexamic acid, aminocaproic acid, and combinations thereof.

In still other embodiments, the methods of treatment may include releasing an antiinflammatory agent, an antifibrotic agent, or a combination thereof, from the deployed drug delivery devices.

Publications cited herein and the materials for which they are cited are specifically incorporated by reference.

In the descriptions provided herein, the terms "includes," "is," "containing," "having," and "comprises" are used in an open-ended fashion, and thus should be interpreted to mean "including, but not limited to." When methods, systems, or devices are claimed or described in terms of "comprising" various components or steps, the methods or systems can also "consist essentially of" or "consist of" the various components or steps, unless stated otherwise.

Various numerical ranges may be disclosed herein. When Applicant discloses or claims a range of any type, Applicant's intent is to disclose or claim individually each possible number that such a range could reasonably encompass, including end points of the range as well as any sub-ranges and combinations of sub-ranges encompassed therein, unless otherwise specified. Moreover, all numerical end points of ranges disclosed herein are approximate. As a representative example, Applicant discloses, in one embodiment, that the drug delivery device has a length of about 1.5 cm to about 3 cm when in a retention shape. This range should be interpreted as encompassing lengths of about 1.5 cm to about 3 cm, and further encompasses "about" each of 1.6 cm, 1.7 cm, 1.8 cm, 1.9 cm, 2 cm, 2.1 cm, 2.2 cm, 2.3 cm, 2.4 cm, 2.5 cm, 2.6 cm, 2.7 cm, 2.8 cm, and 2.9 cm, including any ranges and sub-ranges between any of these values.

The term "about", as used herein, refers to values that are within 5 % of the indicated value. For example, "about 2 cm" would encompass 1.9 cm to 2.1 cm.

Many modifications and other implementations of the disclosure set forth herein will be apparent having the benefit of the teachings presented in the foregoing descriptions and the associated drawings. Therefore, it is to be understood that the disclosure is not to be limited to the specific implementations disclosed and that modifications and other implementations are intended to be included within the scope of the appended claims.

EXAMPLES

The present invention is further illustrated by the following non-limiting examples.

*Example 1 - Drug Delivery Devices*

A gemcitabine-releasing device was produced for *in vitro* testing. Dual material polyurethane tubes were used to construct the systems. The drug reservoir lumen of the drug delivery device of this example contained a gemcitabine hydrochloride powder blend, which included gemcitabine hydrochloride, KOLLIDON® 30 polyvinylpyrrolidone (PVP) (BASF Corp., USA), and CAB-O-SIL® fumed silica (Cabot Corp., USA).

The drug delivery devices had a dual lumen structure that included a substantially circular guidewire lumen and a drug reservoir lumen having a crescent cross-sectional shape, such as the drug reservoir lumen depicted at FIG. 1C. The elastic body of the device was made of a barium sulfate loaded tecoflex polyurethane (TECOFLEX™ EG-80A-B20, 20 % barium sulfate loaded, tecoflex polyurethane, Lubrizol Life Sciences, USA), and a TECOPHILIC™ TPU polyurethane (HP-60D-35, Lubrizol Life Sciences, USA). The tecoflex polyurethane was a water permeable and drug impermeable material with a barium sulfate loading, and the material allowed for visualization during insertion. This material permitted water to diffuse into the drug delivery device, which contributed to dissolution of the on-board drug payload. The TECOPHILIC™ TPU polyurethane was used to form a drug permeable stripe. The TECOPHILIC™ TPU polyurethane, therefore, is a water permeable and drug permeable material that allowed water into the drug delivery device, and allowed the dissolved drug to traverse the drug permeable stripe and enter the area surrounding the drug delivery device.

A first spacer was heat sealed into a first end of the drug reservoir lumen of the drug delivery device. The gemcitabine hydrochloride powder blend was then packed into the drug reservoir lumen of the drug delivery device, and a second spacer was then heat sealed into the second end of the drug reservoir lumen, sealing/closing-off the drug reservoir lumen.

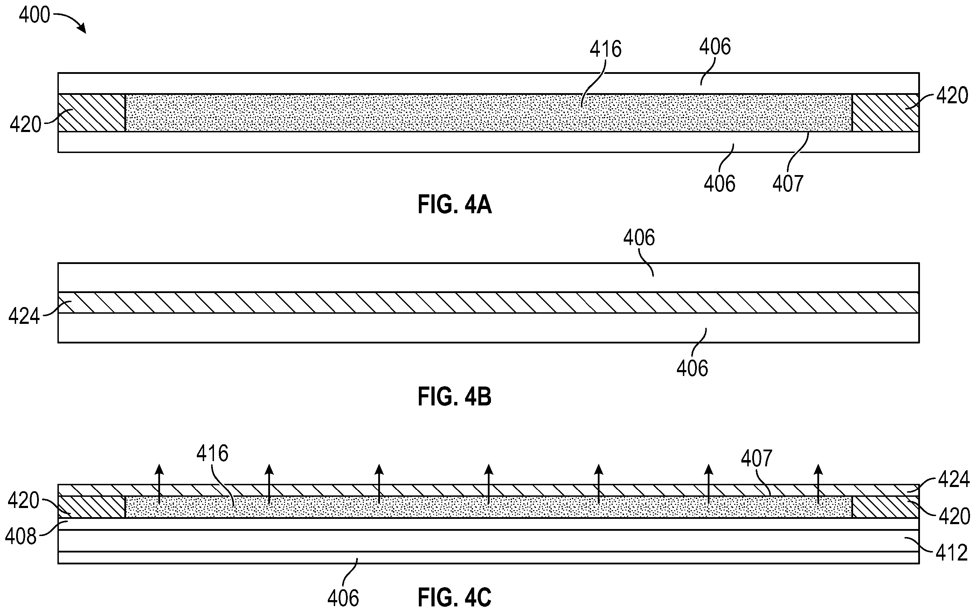

The drug delivery device of this example is shown in **FIGS. 4A-4C.** The drug delivery device **400** included an outer wall **406** formed of a tecoflex polyurethane with a drug permeable stripe **424** of tecophilic polyurethane. Each end of the drug reservoir lumen **407** was sealed with a spacer **420,** and the drug reservoir lumen was loaded with the drug payload **416,** the gemcitabine powder blend. The drug permeable stripe **424** ran along the length of the drug delivery device to provide a path for drug release by transwall diffusion, as illustrated by the arrows passing through the drug permeable stripe **424** in **FIG. 4C. FIG. 4C** also shows the guidewire lumen **412** defined between outer wall **406** and inner wall **408.**

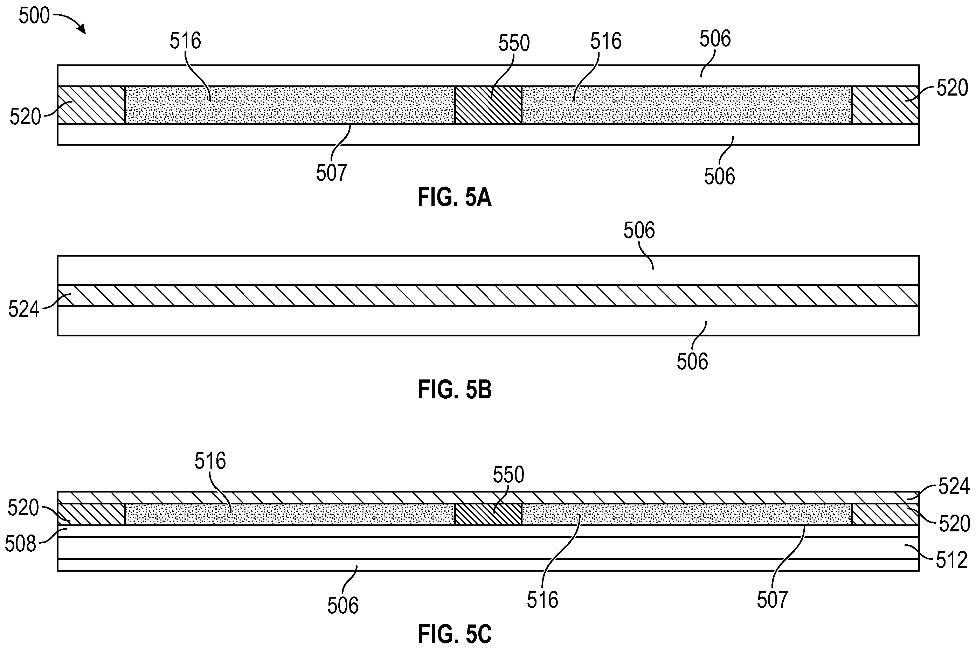

An alternative design of the device was also made, which included the addition of a spacer in the middle of the drug reservoir lumen. This design would be particularly useful when the middle spacer and the end spacers include a radio-opaque material, which would enable a three-point visualization of the device when it is deployed in vivo. In such an embodiment, it would not be necessary to include a radio-opaque material in the material forming the elastic body. An example of such a drug delivery device is shown in **FIGS. 5A-5C.** The drug delivery device **500** included an outer wall **506** formed of a tecoflex polyurethane with a tecophilic polyurethane drug permeable stripe **524.** Each end of the drug reservoir lumen **507** was sealed with a tecoflex polyurethane spacer **520.** The drug reservoir lumen contained the drug payload **516** (the gemcitabine powder blend) and a tecoflex polyurethane spacer **550,** which was disposed approximately midway between the end spacers **520.** The assembly process included (i) packing about half the gemcitabine powder formulation into the drug reservoir lumen after placement of the first spacer, (ii) inserting the middle spacer into the drug reservoir lumen, (iii) packing the remainder of the drug payload into the drug reservoir lumen, and then (iv) installing the second end spacer. The end spacers were heat sealed into the ends of the drug reservoir lumen. The drug permeable stripe **524** ran along the length of the drug delivery device to provide a path for drug release by transwall diffusion, as illustrated by the arrows passing through the drug permeable stripe **524** in **FIG. 5C. FIG. 5C** also shows the guidewire lumen **512** defined between outer wall **506** and inner wall **508.**

After the drug delivery devices were assembled, a retention shape was applied to the devices using a heat setting process: The drug delivery devices were elastically deformed into a retention shape, and then placed into an oven at 90 °C for 10 minutes. The drug delivery devices were left in the retention shape as they cooled to room temperature, and the drug delivery devices maintained the retention shape. Single coil and helical retention shapes are produced.

After the drug delivery devices were imparted with a retention shape, retrieval strings were attached to the ends of the drug delivery devices. The retrieval strings were a monofilament nylon material, which were added to assist in the placement of the drug delivery device, the extraction of the drug delivery device, or both the placement and extraction of the drug delivery device. Alternatively, a monofilament polyethylene material may be used as a retrieval string, due to its ability to maintain its mechanical properties *in vivo.* The retrieval strings passed from the guidewire lumens of the drug delivery devices and through (at a distance of 2.5 mm from the end of the drug delivery devices) the [1] first spacers in the drug reservoir lumens, and [2] the outer wall opposing the drug reservoir lumen. Then the retrieval strings were pulled through until they doubled over, and then the strings were tied together. The retrieval strings were set to a length so that the end could reside in the bladder of the test animal (described below) in order to be easily grasped and removed at the end of treatment.

*Example 2 - In vivo Testing of the Drug Delivery Devices*

The migrations of the two drug delivery devices having different retention shapes were evaluated using *in vivo* testing.

Single coil designs (e.g., those of **FIG. 2**) having lengths of 4 cm and 6 cm (when in a substantially straightened deployment shape) were deployed into the poles of the renal pelvis of kidneys of domestic Yorkshire swine. At necropsy, it was found that the drug delivery devices migrated, and that the smaller drug delivery devices were more prone to migrations than the larger drug delivery devices. Also, the single coil drug delivery devices were shorter in length in order to fit in the available renal pelvis space, which limited drug payload.

A single coil drug delivery device was placed into the upper and the lower pole of four kidneys in four domestic Yorkshire swine. Two small (i.e., 4 cm) single coil drug delivery devices were deployed for 10 days in two animals (four total small drug delivery devices), and two large drug delivery devices (i.e., 6 cm) were deployed for 10 days in one animal. One large drug delivery device (i.e., 6 cm) was deployed for 10 days in the remaining animal, due to the small geometry of the renal pelvis. One of the four small drug delivery devices migrated into the proximal ureter, two small drug delivery devices migrated to the hilum of the kidney, and the remaining small drug delivery device remained in the kidney at the time of necropsy. The three large drug delivery devices were found in the kidneys at the time of necropsy. The small drug delivery devices, therefore, appeared to be more prone to migration than the larger drug delivery devices.

Helical (multi-coil) drug delivery devices having lengths of 6 cm and 8 cm (when in a relatively straightened deployment shape) and an inner coil diameter of 0.5 cm, and helical drug delivery devices having lengths of 10 cm and 12 cm and an inner coil diameter of 1 cm were deployed into the poles of the renal pelvis of kidneys. At necropsy, it was found that the drug delivery devices of all sizes did not exhibit significant migration potential, which indicated an improvement in retention with the helix designs relative to the coil designs. Additionally, it appeared that the longer systems were more secure than the shorter systems after placement. Due to their three-dimensional shape, the multi-coil drug delivery devices touched the walls of the renal pelvis in all directions, thereby increasing retention. The relative complexity of the shape also made these drug delivery devices less likely to travel back out and down the ureter. The longer length of the multi-coil drug delivery devices also allowed a greater drug payload to be used.

The helix designs were subjected to a seven day deployment into the upper pole of eight kidneys in four domestic Yorkshire swine. The deployments of the helix designs of this example are provided in the following table:

| Animal No. | Side | Helix Design Size (cm) |
|---|---|---|
| 1 | Left | 12 |
| 1 | Right | 8 |
| 2 | Left | 10 |
| 2 | Right | 12 |
| 3 | Left | 10 |
| 3 | Right | 12 |
| 4 | Left | 6 |
| 4 | Right | 8 |

After seven days, one of the eight helix design drug delivery devices had migrated into the ureter. The migrating drug delivery device may have been at least partially dislodged during attempts to remove the string fixture. The helix design drug delivery devices of all sizes (length and coil diameter) did not exhibit a significant migration potential, which indicated an overall improvement in retention.

*Example 3 - Drug Delivery Device Shape Variations*

A drug delivery device, as depicted in **FIG. 6,** was produced with three turns in the helical portion and straight ends extending roughly perpendicular to the helical portion of the device. The straight ends were shaped with heat setting, and aligned diagonally across the device. The outer coil diameter was 12 mm.

A drug delivery device was produced as depicted in **FIG. 7,** with six turns in the helical portion and straight ends formed with heat setting. The outer coil diameter of the device was 8 mm. The uncoiled length of the device was 120 mm.

A drug delivery device was produced as depicted in **FIG. 8,** with three coils spaced apart with two intermediate straight sections. The uncoiled length of the device was 85 mm.

A drug delivery device was produced as depicted in **FIG. 9,** with two coils spaced apart by a single intermediate straight section. The uncoiled length of the device was 75 mm.

A drug delivery device with eight turns and straight ends was also produced. The device had an outer coil diameter of 7 mm.

The leading end of each of these devices was heat shaped to provide a tapered end, as depicted in **FIG. 10A.**

*Example 4 - In Vitro Release of Drug*

Several embodiments of the drug delivery devices of Example 1 were tested for *in vitro* release of the gemcitabine. The different embodiments tested in this example included drug delivery devices that [1] had different arc angles of the drug permeable stripes, [2] were made with different retrieval string attachment methods, [3] had different lengths, and [4] had different heat set shapes.

Each drug delivery device was placed in 300 g of deionized water at 37 °C, and time point samples were collected at pre-determined time points to construct *in vitro* release profiles.

a. Stripe Angles

Drug delivery devices having two different stripe arc angles were tested. The stripe arc angle of the first drug delivery device was 60 °, and the stripe arc angle of the second drug delivery device was 120 °.

The smaller stripe arc angle (*i.e.* 60 °) resulted in a longer release duration. The drug delivery device having a 120 ° stripe arc angle had a release profile lasting 4 days, while the drug delivery device having a 60 ° stripe arc angle had a release profile that lasted 7 days.

b. Retrieval string Attachment

The method of retrieval string attachment explained at Example 1 resulted in no leaking, and was the simplest method of attachment tested.

c. Drug Delivery Device Length

Drug delivery devices were tested that had a coil retention shape or a helical retention shape, and lengths of 4 cm (coil retention shape), 8 cm (coil retention shape), 10 cm (helical retention shape), and 12 cm (helical retention shape) in a relatively straightened deployment shape. The drug delivery devices having a length of 4 cm and 8 cm, however, migrated undesirably after deployment. The drug delivery devices having a length of 10 cm or 12 cm did not migrate undesirably after deployment, and the change in length (from 4 cm or 8 cm) did not affect the release duration of the drug delivery device, but the change did increase the peak release rate.

d. Heat Set Configuration

Changes to the heat set configuration had no noticeable effect on the release profile of the drug delivery devices.

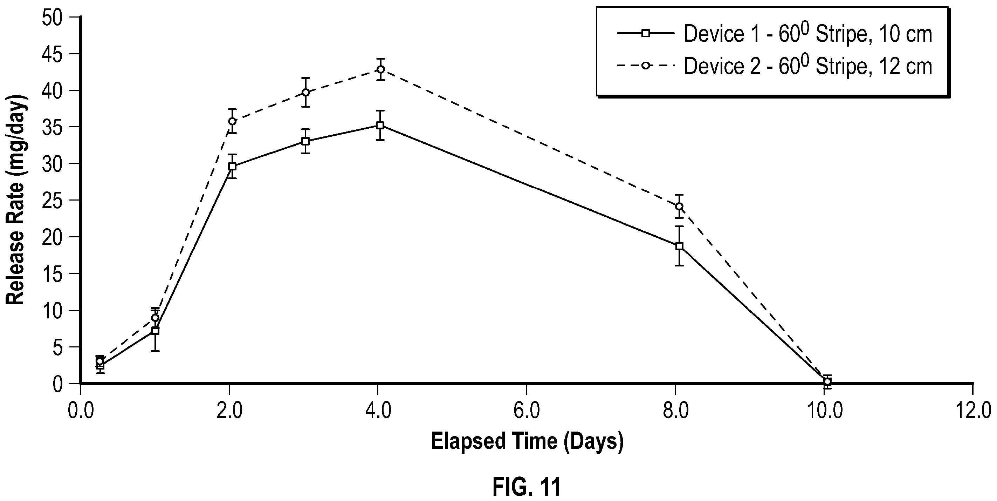

FIG. 11 depicts the release profiles of two different drug delivery devices of this example, the first having a stripe with an arc angle of 60 °, a length of 10 cm when in a relatively straightened deployment shape, and a helix heat set configuration. The second drug delivery device was identical to the first, except for its length, which was 12 cm. The plots of FIG. 11 indicate that the release rates achieved with the two drug delivery devices were similar, but the device having a longer length (Device 2) released a greater amount of the drug on each of days 1-9.

e. Stripe Angle

Changes to the stripe angle had no noticeable effect on the release profile of the drug delivery devices tested when the device with the smaller stripe angle had a larger drug payload.

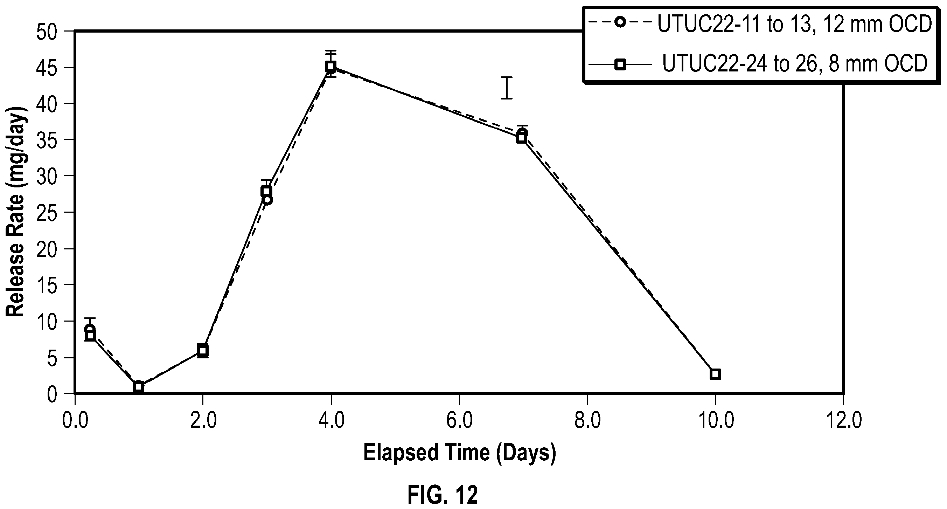

FIG. 12 depicts the release profiles of two different drug delivery device configurations: A first configuration of the drug delivery device was made with EG-100A-B20 as a base material, and a second configuration of the drug delivery device was made with EG-80A-B20 as a base material. EG-100A-B20 is a stiffer material and increased the column strength of the drug delivery device, causing less collapse in the drug delivery device as it was pushed along a guidewire. The first configuration of the drug delivery device had an outer diameter of 9Fr and higher drug payload potential, while the second configuration had an outer diameter of 8Fr. The drug permeable stripe of both drug delivery devices was made of HP-60D-35. The stripe angle of the second configuration was 60°. Because the first configuration has a higher drug payload potential the stripe angle was decreased to 55° to maintain peak release of the drug profile. As seen in FIG. 12, despite modifications to the drug delivery device to produce the second configuration, both configurations had a very similar drug release profile and peak release was maintained.

*Example 5 - Crescent-Shaped Tablets*

Crescent-shaped tablets were produced, including those that correspond to the dimensions of the drug reservoir lumen of the drug delivery devices of Example 1. The crescent-shaped tablets were produced using a single station automated press, and a placebo blend. The crescent-shape tablets resembled the one depicted in FIG. 13.

**Claims**

1. A drug delivery device (100, 200, 300, 400, 500, 600, 700, 800, 900, 1000, 1500, 1600) for deployment in a renal pelvis of a patient, the drug delivery device comprising:

    an elastic body (102, 202, 302), wherein the elastic body comprises

        (i) an outer tube comprising an elongated outer wall, and
        (ii) an elongated, arcuate inner wall (108, 308, 408, 508, 1008, 1008, 1608) located within the outer tube and integrally connected to an inner surface of the outer wall along two opposed edges of the arcuate inner wall, the outer and inner walls together defining (a) a guidewire lumen (112, 312, 412, 512, 1012, 1512, 1612) on a concave side of the inner wall, and (b) a drug reservoir lumen (114, 314, 407) on an opposed convex side of the inner wall, the drug reservoir lumen being closed off at its opposed ends; and

    a drug payload (116, 316, 416, 516) disposed in the drug reservoir lumen, wherein the drug payload comprises at least one drug,
    wherein the drug delivery device is elastically deformable between a deployment shape for passage of the drug delivery device through a ureter and into the renal pelvis of the patient, and a retention shape which is configured to mitigate migration of the device from the renal pelvis, such that the drug delivery device is wholly contained within the renal pelvis, with the optional exception of a retrieval string (180, 280, 1580, 1680, 1902) extending at least into the patient's ureter.

2. The drug delivery device (100, 200, 300, 400, 500, 600, 700, 800, 900, 1000, 1500, 1600) of claim 1, wherein the device (100, 200, 300, 400, 500, 600, 700, 800, 900, 1000, 1500, 1600)is biased to be in the retention shape in the absence of a guidewire inserted into the guidewire lumen.

3. The drug delivery device (100, 200, 300, 400, 500, 600, 700, 800, 900, 1000, 1500, 1600) of claim 1, wherein the retention shape has a helical portion; optionally wherein one of the following options are fulfilled:

   a) the retention shape further comprises straight end portions which extend in the longitudinal direction of the device; and
   b) the helical portion comprises from two to ten turns; optionally wherein at least two of the turns are separated by an intermediate straight portion.

4. The drug delivery device (600, 700, 800, 900, 1000) of any one of the claims 1 to 3,
   wherein one or both ends of the device body is tapered.

5. The drug delivery device (100, 200, 300, 400, 500, 600, 700, 800, 900, 1000, 1500, 1600) of any one of claims 1 to 3, wherein the outer tube comprises two different materials of construction, of which a first material is impermeable to the drug when the drug is in solution and a second material which is permeable to the drug when the drug is in solution; and wherein the second material is adjacent to the drug payload.

6. The drug delivery device (100, 200, 300, 400, 500, 600, 700, 800, 900, 1000, 1500, 1600) of claim 5, wherein the second material is in the form of a drug permeable stripe extending the length of the elongated outer wall; optionally wherein one of the following options is fulfilled:

   a) the outer wall is cylindrical and the drug permeable stripe has an arc angle of about 30 ° to about 120 ° of the circumference of the outer wall in the cross section; optionally wherein the drug permeable stripe has an arc angle of about 55 ° to about 120 ° of the circumference of the outer wall in the cross section;
   b) the first material comprises a tecoflex polyurethane;
   c) the first material further comprises a radio-opaque filler; and
   d) the second material comprises a tecophilic polyurethane.

7. The drug delivery device of any one of claims 1 to 3, wherein one of the following options is fulfilled:

   a) the drug reservoir lumen has a crescent cross-sectional shape; and
   b) the guidewire lumen has a circular cross-sectional shape.

8. The drug delivery device (300) of any one of claims 1 to 3, further comprising a retention frame lumen (330) and a retention frame disposed in the retention frame lumen, the retention frame being an elastic wire configured to bias the drug delivery device into the retention shape.

9. The drug delivery device (100, 200, 300, 400, 500, 600, 700, 800, 900, 1000, 1500, 1600) of any one of claims 1 to 3, wherein the retention shape comprises a helical portion and the drug delivery device has a length of about 5 cm to about 15 cm when in the deployment shape; optionally wherein the drug delivery device has a length of about 0.8 cm to about 2 cm when in the retention shape.

10. The drug delivery device (100, 200, 300, 400, 500, 600, 700, 800, 900, 1000, 1500, 1600) of any one of claims 1 to 3, wherein each of the opposed ends of the drug reservoir lumen is sealed by a spacer and wherein one or more of the following options is fulfilled:

   a) the device further comprises at least one middle spacer disposed in the drug reservoir lumen at a position between the opposed ends; and
   b) the elastic body, the end spacers, and/or the at least one middle spacer comprises a radio-opaque filler material.

11. The drug delivery device of (100, 200, 300, 400, 500, 600, 700, 800, 900, 1000, 1500, 1600) any one of claims 1 to 10, further comprising a retrieval string attached to the drug delivery device; optionally wherein the retrieval string has a length sufficient for an end of the retrieval string to reside in the patient's bladder when the drug delivery device is deployed in the renal pelvis.

12. The drug delivery device (100, 200, 300, 400, 500, 600, 700, 800, 900, 1000, 1500, 1600) of any one of claims 1 to 3, wherein the drug payload is in a solid or a semi-solid form; optionally wherein one of the following options is fulfilled:

a) the drug payload is the form of a powder or a plurality of tablets; and
b) the drug payload is the form of a plurality of tablets which have a crescent cross-sectional shape.

13. The drug delivery device (100, 200, 300, 400, 500, 600, 700, 800, 900, 1000, 1500, 1600) of any one of claims 1 to 3, wherein one of the following options is fulfilled:

a) the outer tube and the inner wall are formed together by a co-extrusion process; and
b) the elastic body is thermally shape set to have the retention shape.

14. A system for administration of a drug to a patient in need thereof, the system comprising:

the drug delivery device of any one of claims 1 to 13; and
a guidewire deployment system (1400) for deploying the drug delivery device in a renal pelvis of the patient, wherein the guidewire deployment system including (i) a guidewire (1402) configured for operable association with the drug delivery device and having a distal end portion capable of extending into the renal pelvis while an opposed proximal end portion extends out of the patient's urethra, and (ii) a plunger device for pushing the drug delivery device over the guidewire and off of the distal end and into the renal pelvis, wherein the guidewire has a cross-sectional area dimensioned to pass through the guidewire lumen of the drug delivery device.

15. The system of claim 14, wherein the plunger device comprises:

a plunger (1404);
a handle;
a sheath extending between the plunger and the handle, the sheath transferring to the plunger a driving force applied to the handle; and

an internal bore for receiving the guidewire, such that the plunger device can travel over the guidewire; optionally wherein the plunger further comprises a stop configured to indicate that the drug delivery device has separated from the guidewire.

**Patentansprüche**

1. Arzneimittelabgabevorrichtung (100, 200, 300, 400, 500, 600, 700, 800, 900, 1000, 1500, 1600) zum Einsetzen in einem Nierenbecken eines Patienten, die Arzneimittelabgabevorrichtung umfassend:

einen elastischen Körper (102, 202, 302), wobei der elastische Körper umfasst

(i) einen äußeren Schlauch, umfassend eine längliche äußere Wand, und
(ii) eine längliche, bogenförmige innere Wand (108, 308, 408, 508, 1008, 1008, 1608), die sich innerhalb des äußeren Schlauchs befindet und integral mit einer inneren Oberfläche der äußeren Wand entlang zweier gegenüberliegender Kanten der bogenförmigen inneren Wand verbunden ist, wobei die äußere und die innere Wand zusammen (a) ein Führungsdrahtlumen (112, 312, 412, 512, 1012, 1512, 1612) an einer konkaven Seite der inneren Wand und (b) ein Arzneimittelreservoirlumen (114, 314, 407) an einer gegenüberliegenden konvexen Seite der inneren Wand definieren, wobei das Arzneimittelreservoirlumen an seinen gegenüberliegenden Enden verschlossen ist; und

eine Arzneimittelnutzlast (116, 316, 416, 516), die in dem Arzneimittelreservoirlumen angeordnet ist, wobei die Arzneimittelnutzlast mindestens ein Arzneimittel umfasst,

wobei die Arzneimittelabgabevorrichtung elastisch verformbar ist zwischen einer Einsatzgestalt, für ein Durchgehen der Arzneimittelabgabevorrichtung durch einen Harnleiter und in das Nierenbecken des Patienten, und einer Rückhaltegestalt, die konfiguriert ist, um eine Migration der Vorrichtung aus dem Nierenbecken zu vermindern, derart, dass die Arzneimittelabgabevorrichtung vollständig innerhalb des Nierenbeckens enthalten ist, mit der optionalen Ausnahme einer Rückholschnur (180, 280, 1580, 1680, 1902), die sich mindestens in den Harnleiter des Patienten erstreckt.

2. Arzneimittelabgabevorrichtung (100, 200, 300, 400, 500, 600, 700, 800, 900, 1000, 1500, 1600) nach Anspruch 1, wobei die Vorrichtung (100, 200, 300, 400, 500, 600, 700, 800, 900, 1000, 1500, 1600) vorgespannt ist, um in der Abwesenheit eines in das Führungsdrahtlumen eingeführten Führungsdrahts in der Rückhaltegestalt vorzuliegen.

3. Arzneimittelabgabevorrichtung (100, 200, 300, 400, 500, 600, 700, 800, 900, 1000, 1500, 1600) nach Anspruch 1, wobei die Rückhaltegestalt einen spiralförmigen Abschnitt aufweist; wobei gegebenenfalls eine der folgenden Optionen erfüllt ist:

a) die Rückhaltegestalt umfasst ferner gerade Endabschnitte, die sich in der Längsrichtung der Vorrichtung erstrecken; und
b) der spiralförmige Abschnitt umfasst von zwei bis zehn Windungen; wobei gegebenenfalls mindestens zwei der Windungen durch einen dazwischenliegenden geraden Abschnitt getrennt sind.

4. Arzneimittelabgabevorrichtung (600, 700, 800, 900, 1000) nach einem der Ansprüche 1 bis 3, wobei ein oder beide Enden des Vorrichtungskörpers verjüngt sind.

5. Arzneimittelabgabevorrichtung (100, 200, 300, 400, 500, 600, 700, 800, 900, 1000, 1500, 1600) nach einem der Ansprüche 1 bis 3, wobei der äußere Schlauch zwei verschiedene Konstruktionsmaterialien umfasst, von denen ein erstes Material undurchlässig für das Arzneimittel ist, wenn das Arzneimittel in Lösung vorliegt, und ein zweites Material durchlässig für das Arzneimittel ist, wenn das Arzneimittel in Lösung vorliegt; und wobei das zweite Material an die Arzneimittelnutzlast angrenzt.

6. Arzneimittelabgabevorrichtung (100, 200, 300, 400, 500, 600, 700, 800, 900, 1000, 1500, 1600) nach Anspruch 5, wobei das zweite Material in der Form eines arzneimitteldurchlässigen Streifens vorliegt, der sich über die Länge der länglichen äußeren Wand erstreckt; wobei gegebenenfalls eine der folgenden Optionen erfüllt ist:

a) die äußere Wand ist zylindrisch und der arzneimitteldurchlässige Streifen weist einen Bogenwinkel von etwa 30° bis etwa 120° des Umfangs der äußeren Wand im Querschnitt auf; wobei gegebenenfalls der arzneimitteldurchlässige Streifen einen Bogenwinkel von etwa 55° bis etwa 120° des Umfangs der äußeren Wand im Querschnitt aufweist;
b) das erste Material umfasst ein Tecoflex-Polyurethan;
c) das erste Material umfasst ferner einen röntgendichten Füllstoff; und
d) das zweite Material umfasst ein Tecophilic-Polyurethan.

7. Arzneimittelabgabevorrichtung nach einem der Ansprüche 1 bis 3, wobei eine der folgenden Optionen erfüllt ist:

a) das Arzneimittelreservoirlumen weist eine sichelförmige Querschnittsgestalt auf; und
b) das Führungsdrahtlumen weist eine kreisförmige Querschnittsgestalt auf.

8. Arzneimittelabgabevorrichtung (300) nach einem der Ansprüche 1 bis 3, ferner umfassend ein Rückhalterahmenlumen (330) und einen in dem Rückhalterahmenlumen angeordneten Rückhalterahmen, wobei der Rückhalterahmen ein elastischer Draht ist, der konfiguriert ist, um die Arzneimittelabgabevorrichtung in die Rückhaltegestalt vorzuspannen.

9. Arzneimittelabgabevorrichtung (100, 200, 300, 400, 500, 600, 700, 800, 900, 1000, 1500, 1600) nach einem der Ansprüche 1 bis 3, wobei die Rückhaltegestalt einen spiralförmigen Abschnitt umfasst und die Arzneimittelabgabevorrichtung eine Länge von etwa 5 cm bis etwa 15 cm aufweist, wenn sie in der Einsatzgestalt vorliegt; wobei gegebenenfalls die Arzneimittelabgabevorrichtung eine Länge von etwa 0,8 cm bis etwa 2 cm aufweist, wenn sie in der Rückhaltegestalt vorliegt.

10. Arzneimittelabgabevorrichtung (100, 200, 300, 400, 500, 600, 700, 800, 900, 1000, 1500, 1600) nach einem der Ansprüche 1 bis 3, wobei jedes der gegenüberliegenden Enden des Arzneimittelreservoirlumens durch einen Abstandshalter abgedichtet ist und wobei eine oder mehrere der folgenden Optionen erfüllt ist:

a) die Vorrichtung umfasst ferner mindestens einen mittleren Abstandshalter, der in dem Arzneimittelreservoirlumen an einer Position zwischen den gegenüberliegenden Enden angeordnet ist; und
b) der elastische Körper, die Endabstandshalter und/oder der mindestens eine mittlere Abstandshalter umfassen ein röntgendichtes Füllmaterial.

11. Arzneimittelabgabevorrichtung (100, 200, 300, 400, 500, 600, 700, 800, 900, 1000, 1500, 1600) nach einem der Ansprüche 1 bis 10, ferner umfassend eine Rückholschnur, die an der Arzneimittelabgabevorrichtung befestigt ist; wobei gegebenenfalls die Rückholschnur eine Länge aufweist, die ausreicht, damit ein Ende der Rückholschnur in der Blase des Patienten verbleibt, wenn die Arzneimittelabgabevorrichtung in dem Nierenbecken eingesetzt ist.

12. Arzneimittelabgabevorrichtung (100, 200, 300, 400, 500, 600, 700, 800, 900, 1000, 1500, 1600) nach einem der Ansprüche 1 bis 3, wobei die Arzneimittelnutzlast in einer festen oder einer halbfesten Form vorliegt; wobei gegebenenfalls eine der folgenden Optionen erfüllt ist:

a) die Arzneimittelnutzlast liegt in der Form eines Pulvers oder einer Vielzahl von Tabletten vor; und
b) die Arzneimittelnutzlast liegt in der Form einer Vielzahl von Tabletten vor, die eine sichelförmige Querschnittsgestalt aufweisen.

13. Arzneimittelabgabevorrichtung (100, 200, 300, 400, 500, 600, 700, 800, 900, 1000, 1500, 1600) nach einem der Ansprüche 1 bis 3, wobei eine der folgenden Optionen erfüllt ist:

a) der äußere Schlauch und die innere Wand werden durch ein Koextrusionsverfahren zusammen ausgebildet; und
b) der elastische Körper ist in seiner Gestalt thermisch fixiert, um die Rückhaltegestalt aufzuweisen.

14. System zum Verabreichen eines Arzneimittels an einen Patienten, der dies benötigt, das System umfassend:

die Arzneimittelabgabevorrichtung nach einem der Ansprüche 1 bis 13; und
ein Führungsdrahteinsatzsystem (1400) zum Einsetzen der Arzneimittelabgabevorrichtung in ein Nierenbecken des Patienten, das Führungsdrahteinsatzsystem einschließlich (i) eines Führungsdrahts (1402), der für eine betriebsfähige Verknüpfung mit der Arzneimittelabgabevorrichtung konfiguriert ist und einen distalen Endabschnitt aufweist, der in der Lage ist, sich in das Nierenbecken zu erstrecken, während sich ein gegenüberliegender proximaler Endabschnitt aus der Harnröhre des Patienten heraus erstreckt, und (ii) einer Stempelvorrichtung zum Schieben der Arzneimittelabgabevorrichtung über den Führungsdraht und von dem distalen Ende ab und in das Nierenbecken hinein,
wobei der Führungsdraht eine Querschnittsfläche aufweist, die dimensioniert ist, um durch das Führungsdrahtlumen der Arzneimittelabgabevorrichtung hindurchzugehen.

15. System nach Anspruch 14, wobei die Stempelvorrichtung umfasst:

einen Stempel (1404);
einen Griff;
eine Hülle, die sich zwischen dem Stempel und dem Griff erstreckt, wobei die Hülle eine auf den Griff ausgeübte Antriebskraft auf den Stempel überträgt; und
eine interne Bohrung zum Aufnehmen des Führungsdrahts, derart, dass sich die Stempelvorrichtung über den Führungsdraht bewegen kann; wobei gegebenenfalls der Stempel ferner einen Anschlag umfasst, der konfiguriert ist, um anzuzeigen, dass sich die Arzneimittelabgabevorrichtung von dem Führungsdraht getrennt hat.

**Revendications**

1. Dispositif de délivrance de médicament (100, 200, 300, 400, 500, 600, 700, 800, 900, 1000, 1500, 1600) pour déploiement dans un bassinet rénal d'un patient, le dispositif de délivrance de médicament comprenant :

un corps élastique (102, 202, 302), dans lequel le corps élastique comprend

(i) un tube externe comprenant une paroi externe allongée, et

(ii) une paroi interne arquée (108, 308, 408, 508, 1008, 1008, 1608) allongée, localisée au sein du tube externe et reliée de façon solidaire à une surface interne de la paroi externe le long de deux bords opposés de la paroi interne arquée, les parois externe et interne définissant ensemble (a) une lumière de fil-guide (112, 312, 412, 512, 1012, 1512, 1612) sur un côté concave de la paroi interne, et (b) une lumière de réservoir de médicament (114, 314, 407) sur un côté convexe opposé de la paroi interne, la lumière de réservoir de médicament étant obturée au niveau de ses extrémités opposées ; et

une charge utile de médicament (116, 316, 416, 516) disposée dans la lumière de réservoir de médicament, dans lequel la charge utile de médicament comprend au moins un médicament,

dans lequel le dispositif de délivrance de médicament est élastiquement déformable entre une forme de déploiement pour le passage du dispositif de délivrance de médicament à travers un uretère et dans le bassinet rénal du patient, et une forme de rétention qui est conçue pour atténuer une migration du dispositif par rapport au bassinet rénal, de telle sorte que le dispositif de délivrance de médicament est entièrement contenu au sein du bassinet rénal, à l'exception facultative d'un cordon de récupération (180, 280, 1580, 1680, 1902) s'étendant au moins dans l'uretère du patient.

2. Dispositif de délivrance de médicament (100, 200, 300, 400, 500, 600, 700, 800, 900, 1000, 1500, 1600) selon la revendication 1, dans lequel le dispositif (100, 200, 300, 400, 500, 600, 700, 800, 900, 1000, 1500, 1600) est sollicité pour être dans la forme de rétention en l'absence d'un fil-guide inséré dans la lumière de fil-guide.

3. Dispositif de délivrance de médicament (100, 200, 300, 400, 500, 600, 700, 800, 900, 1000, 1500, 1600) selon la revendication 1, dans lequel la forme de rétention a une partie hélicoïdale ; facultativement dans lequel l'une des options suivantes est respectée :

a) la forme de rétention comprend en outre des parties d'extrémité linéaires qui s'étendent dans la direction longitudinale du dispositif ; et

b) la partie hélicoïdale comprend de deux à dix spires ; facultativement dans lequel au moins deux des spires sont séparées par une partie linéaire intermédiaire.

4. Dispositif de délivrance de médicament (600, 700, 800, 900, 1000) selon l'une quelconque des revendications 1 à 3, dans lequel l'une et/ou l'autre des extrémités du corps de dispositif sont effilées.

5. Dispositif de délivrance de médicament (100, 200, 300, 400, 500, 600, 700, 800, 900, 1000, 1500, 1600) selon l'une quelconque des revendications 1 à 3, dans lequel le tube externe comprend deux matériaux de construction différents, dont un premier matériau est imperméable au médicament lorsque le médicament est en solution et un second matériau est perméable au médicament lorsque le médicament est en solution ; et dans lequel le second matériau est adjacent à la charge utile de médicament.

6. Dispositif de délivrance de médicament (100, 200, 300, 400, 500, 600, 700, 800, 900, 1000, 1500, 1600) selon la revendication 5, dans lequel le second matériau est sous la forme d'une bande perméable au médicament s'étendant sur la longueur de la paroi externe allongée ; facultativement dans lequel l'une des options suivantes est respectée :

a) la paroi externe est cylindrique et la bande perméable au médicament a un angle d'arc d'environ 30° à environ 120° de la circonférence de la paroi externe dans la coupe transversale ; facultativement dans lequel la bande perméable au médicament a un angle d'arc d'environ 55° à environ 120° de la circonférence de la paroi externe dans la coupe transversale ;

b) le premier matériau comprend un polyuréthane Tecoflex ;

c) le premier matériau comprend en outre une charge radio-opaque ; et

d) le second matériau comprend un polyuréthane Tecophilic.

7. Dispositif de délivrance de médicament selon l'une quelconque des revendications 1 à 3, dans lequel l'une des options suivantes est respectée :

a) la lumière de réservoir de médicament a une forme en coupe transversale en croissant ; et

b) la lumière de fil-guide a une forme en coupe transversale circulaire.

8. Dispositif de délivrance de médicament (300) selon l'une quelconque des revendications 1 à 3, comprenant en outre une lumière de cadre de rétention (330) et un cadre de rétention disposé dans la lumière de cadre de rétention, le cadre de rétention étant un fil élastique conçu pour solliciter le dispositif de délivrance de médicament dans la forme de rétention.

9. Dispositif de délivrance de médicament (100, 200, 300, 400, 500, 600, 700, 800, 900, 1000, 1500, 1600) selon l'une quelconque des revendications 1 à 3, dans lequel la forme de rétention comprend une partie hélicoïdale et le dispositif de délivrance de médicament a une longueur d'environ 5 cm à environ 15 cm lorsqu'il est dans la forme de déploiement ; facultativement dans lequel le dispositif de délivrance de médicament a une longueur d'environ 0,8 cm à environ 2 cm lorsqu'il est dans la forme de rétention.

10. Dispositif de délivrance de médicament (100, 200, 300, 400, 500, 600, 700, 800, 900, 1000, 1500, 1600) selon l'une quelconque des revendications 1 à 3, dans lequel chacune des extrémités opposées de la lumière de réservoir de médicament est scellée par un espaceur et dans lequel une ou plusieurs des options suivantes sont respectées :

   a) le dispositif comprend en outre au moins un espaceur médian disposé dans la lumière de réservoir de médicament au niveau d'une position entre les extrémités opposées ; et
   b) le corps élastique, les espaceurs d'extrémité et/ou l'au moins un espaceur médian comprennent un matériau de charge radio-opaque.

11. Dispositif de délivrance de médicament (100, 200, 300, 400, 500, 600, 700, 800, 900, 1000, 1500, 1600) selon l'une quelconque des revendications 1 à 10, comprenant en outre un cordon de récupération fixé au dispositif de délivrance de médicament ; facultativement dans lequel le cordon de récupération a une longueur suffisante pour qu'une extrémité du cordon de récupération se trouve dans la vessie du patient lorsque le dispositif de délivrance de médicament est déployé dans le bassinet rénal.

12. Dispositif de délivrance de médicament (100, 200, 300, 400, 500, 600, 700, 800, 900, 1000, 1500, 1600) selon l'une quelconque des revendications 1 à 3, dans lequel la charge utile de médicament est sous une forme solide ou semi-solide ; facultativement dans lequel l'une des options suivantes est respectée :

   a) la charge utile de médicament a la forme d'une poudre ou d'une pluralité de comprimés ; et
   b) la charge utile de médicament a la forme d'une pluralité de comprimés qui ont une forme en coupe transversale en croissant.

13. Dispositif de délivrance de médicament (100, 200, 300, 400, 500, 600, 700, 800, 900, 1000, 1500, 1600) selon l'une quelconque des revendications 1 à 3, dans lequel l'une des options suivantes est respectée :

   a) le tube externe et la paroi interne sont formés ensemble par un procédé de coextrusion ; et
   b) le corps élastique est thermiquement mis en forme pour avoir la forme de rétention.

14. Système destiné à l'administration d'un médicament à un patient qui en a besoin, le système comprenant :

   le dispositif de délivrance de médicament selon l'une quelconque des revendications 1 à 13 ; et
   un système de déploiement de fil-guide (1400) permettant de déployer le dispositif de délivrance de médicament dans un bassinet rénal du patient, dans lequel le système de déploiement de fil-guide comporte (i) un fil-guide (1402) conçu pour une association fonctionnelle avec le dispositif de délivrance de médicament et ayant une partie d'extrémité distale capable de s'étendre dans le bassinet rénal alors qu'une partie d'extrémité proximale opposée s'étend à l'extérieur de l'urètre du patient, et (ii) un dispositif de piston permettant de pousser le dispositif de délivrance de médicament par-dessus le fil-guide et hors de l'extrémité distale et dans le bassinet rénal, dans lequel le fil-guide a une aire en coupe transversale dimensionnée pour passer à travers la lumière de fil-guide du dispositif de délivrance de médicament.

15. Système selon la revendication 14, dans lequel le dispositif de piston comprend :

   un piston (1404) ;
   une poignée ;
   une gaine s'étendant entre le piston et la poignée, la gaine transférant au piston une force d'entraînement appliquée à la poignée ; et
   un alésage interne permettant de recevoir le fil-guide, de telle sorte que le dispositif de piston peut se déplacer par-dessus le fil-guide ; facultativement dans lequel le piston comprend en outre un arrêt conçu pour indiquer que le dispositif de délivrance de médicament s'est séparé du fil-guide.

FIG. 1A

FIG. 1B

100

112    108

102    104

122

106    114

116

α    124

**FIG. 1C**

200

280

202

**FIG. 2**

**FIG. 3**

FIG. 4A

FIG. 4B

FIG. 4C

EP 3 877 034 B1

FIG. 5A

FIG. 5B

FIG. 5C

EP 3 877 034 B1

600

674

672

676

672

676

**FIG. 6**

700

774

776

776

772

772

**FIG. 7**

800

874

874

874

876

878

878

876

**FIG. 8**

**FIG. 9**

**FIG. 10A**

FIG. 10B

FIG. 11

FIG. 12

EP 3 877 034 B1

**FIG. 13**

**FIG. 14A**

**FIG. 14B**

**FIG. 15**

**FIG. 16**

EP 3 877 034 B1

1780
1784
1782
1720

**FIG. 17**

1800

Start

1802 — Inserting a deployment instrument into a body

1804 — Associating a device with the deployment instrument

1806 — Driving the device into the body

1808 — Removing the deployment instrument from the body

1810 — Retaining the device in the body

1812 — Removing the device from the body

End

**FIG. 18**

**FIG. 19**

**REFERENCES CITED IN THE DESCRIPTION**

**Patent documents cited in the description**

- US 62757798
- US 2016287369 A1
- US 2004215169 A1
- WO 2017132372 A1
- US 2017165460 A1

**Non-patent literature cited in the description**

- **GUARRACINO, F.** On the analysis of cylindrical tubes under flexure: theoretical formulations, experimental data and finite element analyses. *Thin Wall Struct;*, 2003, vol. 41, 127-147